# EUROPEAN PATENT APPLICATION

(11) **EP 3 115 012 A1**
(43) Date of publication of application: **11.01.2017**
(21) Application number: 15758398.0
(22) Date of filing: 02.03.2015
(51) Int. Cl.: A61B 18/12

(54) **TREATMENT INSTRUMENT FOR ENDOSCOPE**

(30) Priority: 04.03.2014 JP 2014042051
(71) Applicant: Olympus Corporation, Hachioji-shi, Tokyo 192-8507 (JP)
(72) Inventor: KOBAYASHI, Tsukasa, Tokyo 192-8507 (JP); SAKAMOTO, Yuji, Tokyo 192-8507 (JP); MORISHITA, Hiroyuki, Tokyo 192-8507 (JP)
(74) Representative: Schicker, Silvia
(86) International application number: PCT/JP2015/056115
(87) International publication number: WO 2015/133442

(57) **Abstract**

A treatment tool for an endoscope includes a sheath; a pre-curved portion which is provided at a distal portion of the sheath, and has a restoring force to restore to a curved shape in which the sheath is bent along a virtual plane including the center axis of the sheath; a knife wire lumen which has a center axis at a position spaced from the virtual plane at the distal portion of the pre-curved portion, and is formed along the center axis of the sheath; a first communication hole which is open in a direction outwardly away from the position of the knife wire lumen in a radial direction with respect to the center axis of the sheath, and communicates an outer circumferential surface positioned at an inward side of the curved shape of the pre-curved portion with the knife wire lumen; a second communication hole which is open in a direction outwardly away from the position of the knife wire lumen in the radial direction with respect to the center axis of the sheath, and communicates the outer circumferential surface positioned at the inward side of the curved shape of the pre-curved portion with the knife wire lumen at a more proximal position of the pre-curved portion than the first communication hole; a wire-shaped cutting portion which protrudes from the first communication hole and the second communication hole and extends between the first communication hole and the second communication hole, the cutting portion being capable of incising tissues; a fixing portion which is provided at a distal end portion of the cutting portion, and provided to fix the cutting portion and the knife wire lumen in a state that the fixing portion is inserted into the knife wire lumen; a guide wire accommodation portion which is formed along a longitudinal axis of the sheath at a position spaced from the knife wire lumen in a circumferential direction around the center axis of the sheath, and into which a guide wire is capable of being inserted; a proximal slit portion which is formed to communicate the guide wire accommodation portion and the outer circumferential surface of the sheath in a region more proximal than a proximal end of the pre-curved portion; a distal slit portion which is formed to communicate an outer circumferential surface at an outward side of the curved shape of the pre-curved portion with the guide wire accommodation portion in a region between the proximal end of the pre-curved portion to an intermediate portion of the pre-curved portion in a direction toward a distal end side of the pre-curved portion; and a high-rigidity region which extends from a distal end of the sheath to a distal end of the distal slit portion, and has a higher torsional rigidity than that of a region more proximal than the proximal end of the pre-curved portion.

## Description

### [Technical Field]

The present invention relates to a treatment tool for an endoscope.

Priority is claimed on Japanese Patent Application No. 2014-042051, filed on March 4, 2014, the content of which is incorporated herein by reference.

### [Background Art]

As a procedure for incising the sphincter of a duodenal papilla portion while observing a duodenal papilla using an endoscope apparatus, endoscopic sphincterotomy (EST) is known. For example, a treatment tool which is used in the EST is disclosed in PTL 1 to PTL 4. PTL 1 discloses a guide wire insertion tool in which a funnel-shaped extension portion is provided, which communicates with a lumen of a catheter in order to easily insert a guide wire into the lumen of the catheter. PTL 2 discloses a high-frequency knife in which a guide arm portion is formed on a knife wire, and a cutting portion of the knife wire can be directed to a desired direction by disposing the guide arm portion in a slit which is formed in a sheath. PTL 3 discloses a treatment tool which can safely perform the EST by providing a cutting portion which is not insulated, and an insulation portion which is insulated in a portion except for the cutting portion, on a distal end portion of a high frequency knife wire. PTL 4 discloses a bile duct treatment catheter which includes a groove which communicates with a guide wire lumen from a position outside a catheter shaft and extends in a longitudinal direction of the shaft so as to easily replace a guide wire.

### [Citation List]

### [Patent Literature]

[PTL 1] United States Patent No. 6606515
[PTL 2] Japanese Unexamined Patent Application, First Publication No. 2001-070316
[PTL 3] Japanese Unexamined Patent Application, First Publication No. 2000-237202
[PTL 4] Published Japanese Translation No. 2001-511023 of the PCT International Publication

### [Summary of Invention]

### [Technical Problem]

As a procedure for incising the sphincter of a duodenal papilla while observing the duodenal papilla using an endoscope apparatus, endoscopic sphincterotomy (EST) is known. For example, PTL 3 discloses the treatment tool optimized for the EST. However, in a case where the knife wire disclosed in the PTL 3 is used, in order to decrease an amount of bleeding in the EST, it is preferable to perform incision at a position shifted in an eleven o'clock direction from a twelve o'clock direction in an endoscopic image, and in the treatment tool disclosed in PTL 3, a complicated operation is required so as to hold the knife wire at the position.

The present invention is made in consideration of the above-described circumstances, and an object thereof is to provide a treatment tool for an endoscope and an incision system capable of stably realizing incision of a papilla while decreasing an amount of bleeding.

### [Solution to Problem]

According to a first aspect of the present invention, a treatment tool for an endoscope includes a sheath which has a center axis along a longitudinal axis; a pre-curved portion which is disposed at a distal portion of the sheath, and has a restoring force to restore to a curved shape in which the sheath is curved along a virtual plane including the center axis of the sheath; a knife wire lumen which has a center axis at a position spaced from the virtual plane at the distal portion of the pre-curved portion, and is formed along the center axis of the sheath; a first communication hole which is open in a direction outwardly away from the position of the knife wire lumen in a radial direction with respect to the center axis of the sheath, and communicates an outer circumferential surface positioned at an inward side of the curved shape of the pre-curved portion with the knife wire lumen; a second communication hole which is open in a direction outwardly away from the position of the knife wire lumen in the radial direction with respect to the center axis of the sheath, and communicates the outer circumferential surface positioned at the inward side of the curved shape of the pre-curved portion with the knife wire lumen at a more proximal position of the pre-curved portion than the first communication hole; a wire-shaped cutting portion which protrudes from the first communication hole and the second communication hole and extends between the first communication hole and the second communication hole, the cutting portion being capable of incising tissues; a fixing portion which is disposed at a distal end portion of the cutting portion, and disposed to fix the cutting portion and the knife wire lumen in a state that the fixing portion is inserted into the knife wire lumen; a guide wire accommodation portion which is formed along a longitudinal axis of the sheath at a position spaced from the knife wire lumen in a circumferential direction around the center axis of the sheath, and into which a guide wire is capable of being inserted; a proximal slit portion which is formed to communicate from the guide wire accommodation portion to the outer circumferential surface of the sheath, the proximal slit portion positioned proximally to a proximal end of the pre-curved portion; a distal slit portion which is formed to communicate an outer circumferential surface at an outward side of the curved shape of the pre-curved portion with the guide wire accommodation portion from the proximal end of the pre-curved portion to an intermediate portion of the pre-curved portion in a direction toward a distal end side of the pre-curved portion; and a high-rigidity region which extends from a distal end of the sheath to a distal end of the distal slit portion, and has a higher torsional rigidity than that of a region more proximal than the proximal end of the pre-curved portion.

According to a second aspect of the present invention, in the treatment tool for an endoscope according to the first aspect, an inlet portion may be formed such that an inner portion of the guide wire accommodation portion is communicated with the outer circumferential surface of the sheath at a proximal end side of the sheath, the inlet portion being capable of being inserted by that the guide wire, and the proximal slit portion may be continuously formed from the proximal end of the pre-curved portion to the inlet portion.

According to a third aspect of the present invention, a treatment tool for an endoscope includes a sheath which has a center axis along a longitudinal axis; a pre-curved portion which is disposed at a distal portion of the sheath, and has a restoring force to restore to a curved shape in which the sheath is curved along a virtual plane including the center axis of the sheath; a knife wire lumen which has a center axis at a position spaced from the virtual plane at the distal portion of the pre-curved portion, and is formed along the center axis of the sheath; a first communication hole which is open in a direction outwardly away from the position of the knife wire lumen in a radial direction with respect to the center axis of the sheath, and communicates an outer circumferential surface positioned at an inward side of the curved shape of the pre-curved portion with the knife wire lumen; a second communication hole which is open in a direction outwardly away from the position of the knife wire lumen in the radial direction with respect to the center axis of the sheath, and communicates the outer circumferential surface positioned at the inward side of the curved shape of the pre-curved portion with the knife wire lumen at a more proximal position of the pre-curved portion than the first communication hole; a wire-shaped cutting portion which protrudes from the first communication hole and the second communication hole and extends between the first communication hole and the second communication hole, the cutting portion being capable of incising tissues; a fixing portion which is provided at a distal end portion of the cutting portion, and provided to fix the cutting portion and the knife wire lumen in a state that the fixing portion is inserted into the knife wire lumen; a guide wire accommodation portion which is formed along a longitudinal axis of the sheath at a position spaced from the knife wire lumen in a circumferential direction around the center axis of the sheath, and into which a guide wire is capable of being inserted; and a distal slit portion which is formed along the center axis of the sheath such that an outer circumferential surface at an outward side of the curved shape of the pre-curved portion is communicated with the guide wire accommodation portion in a region between the proximal end of the pre-curved portion to an intermediate portion of the pre-curved portion in a direction toward a distal end side of the pre-curved portion.

According to a fourth aspect of the present invention, in the treatment tool for an endoscope according to the third aspect, a proximal region of the pre-curved portion including the distal slit portion may have a rigidity such that the proximal region of the pre-curved portion including the distal slit portion is deformed to cause a contour shape of the proximal region of the pre-curved portion including the distal slit portion to be changed from a circular shape to an elliptical shape in a cross section orthogonal to the center axis of the sheath, the proximal region of the pre-curved portion including the distal slit portion may be deformed by receiving a force from an inner wall of a treatment tool channel which is bent by a bendable portion of the endoscope apparatus.

### [Advantageous Effects of Invention]

According to the present invention, it is possible to stably perform incision while decreasing an amount of bleeding in the EST.

### [Brief Description of Drawings]

FIG. 1 is an overall view of an incision system including a treatment tool for an endoscope according to a first embodiment of the present invention.
FIG. 2 is a plan view of the treatment tool for an endoscope according to the first embodiment of the present invention.
FIG. 3A is a sectional view taken along line III-III of FIG. 2.
FIG. 3B is a plan view showing a distal end portion of a sheath of the treatment tool for an endoscope according to the first embodiment of the present invention.
FIG. 4 is a sectional view taken along line IV-IV of FIG. 2.
FIG. 5A is a perspective view showing a portion of the sheath in the treatment tool for an endoscope according to the first embodiment of the present invention.
FIG. 5B is a plan view showing the sheath in the treatment tool for an endoscope according to the first embodiment of the present invention.
FIG. 6 is a plan view showing a portion of an operation portion of the treatment tool for an endoscope according to the first embodiment of the present invention.
FIG. 7 is a partial sectional view showing the sheath and a distal configuration portion of the operation portion in the treatment tool for an endoscope according to the first embodiment of the present invention.
FIG. 8 is a partial sectional view showing a first port portion of the treatment tool for an endoscope according to the first embodiment of the present invention, and is a view when viewed from a direction of line VIII-VIII shown in FIG. 6.
FIG. 9A is a sectional view taken along line IX-IX of FIG. 6.
FIG. 9B is a perspective view showing the first port portion of the treatment tool for an endoscope according to the first embodiment of the present invention.
FIG. 10 is a sectional view showing the distal end portion of the sheath of the treatment tool for an endoscope according to the first embodiment of the present invention on a first virtual plane.
FIG. 11 is a view when the distal portion of the sheath of the treatment tool for an endoscope according to the first embodiment of the present invention is viewed from a direction perpendicular to a second virtual plane.
FIG. 12A is a sectional view taken along line XII-XII of FIG. 11.
FIG. 12B shows a modification example of the sheath of the treatment tool for an endoscope according to the first embodiment of the present invention, and is a sectional view at the same position as line XII-XII of FIG. 11.
FIG. 13 is a sectional view taken along line XIII-XIII of FIG. 11.
FIG. 14 is a view showing the distal portion of the sheath of the treatment tool for an endoscope according to the first embodiment of the present invention, and is a view which includes a partial sectional view of the sheath when viewed from line XIV-XIV shown in FIG. 12A.
FIG. 15 is a view showing a positional relationship between the first port and the operation portion in a state where the treatment tool for an endoscope according to the first embodiment of the present invention is attached to an endoscope apparatus.
FIG. 16 is a view showing a positional relationship between a hook and the first port in a state where the treatment tool for an endoscope according to the first embodiment of the present invention is attached to the endoscope apparatus.
FIG. 17 is a perspective view showing a treatment tool attachment-assisting instrument which can be attached to the endoscope apparatus according to the first embodiment of the present invention.
FIG. 18 is a partial sectional view showing an internal structure of the treatment tool attachment-assisting instrument shown in FIG. 17.
FIG. 19 is a view showing a process when the treatment tool for an endoscope according to the first embodiment of the present invention is used.
FIG. 20 is a view when the state where the treatment tool for an endoscope according to the first embodiment of the present invention is attached to the endoscope apparatus is viewed from a viewpoint of an operator of the endoscope apparatus.
FIG. 21 is a view showing a use aspect of the treatment tool for an endoscope according to the first embodiment of the present invention.
FIG. 22 is a sectional view showing a state where a soft region of the sheath is positioned on the inner surface of a treatment tool channel at a bendable portion.
FIG. 23 is a schematic view showing the treatment tool for an endoscope which is reflected on an endoscopic image which is imaged using the endoscope apparatus according to the first embodiment of the present invention.
FIG. 24 is a schematic view showing an endoscopic image in a process of treatment using the treatment tool for an endoscope according to the first embodiment of the present invention.
FIG. 25 is a view showing an aspect in which a guide wire is attached to the treatment tool for an endoscope according to the first embodiment of the present invention, and is a partial sectional view when viewed from line VIII-VIII shown in FIG. 6.
FIG. 26 is a view showing another example in which the guide wire is attached to the treatment tool for an endoscope according to the first embodiment of the present invention, and is a partial sectional view when viewed from line VIII-VIII shown in FIG. 6.
FIG. 27 is a view showing a process when the treatment tool for an endoscope according to the first embodiment of the present invention is used.
FIG. 28 is a view showing a process in which the treatment tool for an endoscope is removed from the endoscope apparatus in a state where the guide wire which is attached to the treatment tool for an endoscope according to the first embodiment of the present invention remains.
FIG. 29 is a view showing a process in which the sheath and the guide wire of the treatment tool for an endoscope according to the first embodiment of the present invention are separated from each other.
FIG. 30 is a view showing an example of treatment which is performed after the removal of the treatment tool for an endoscope according to the first embodiment of the present invention.
FIG. 31 is a schematic view showing a modification example of the sheath of the first embodiment of the present invention.

### [Description of Embodiments]

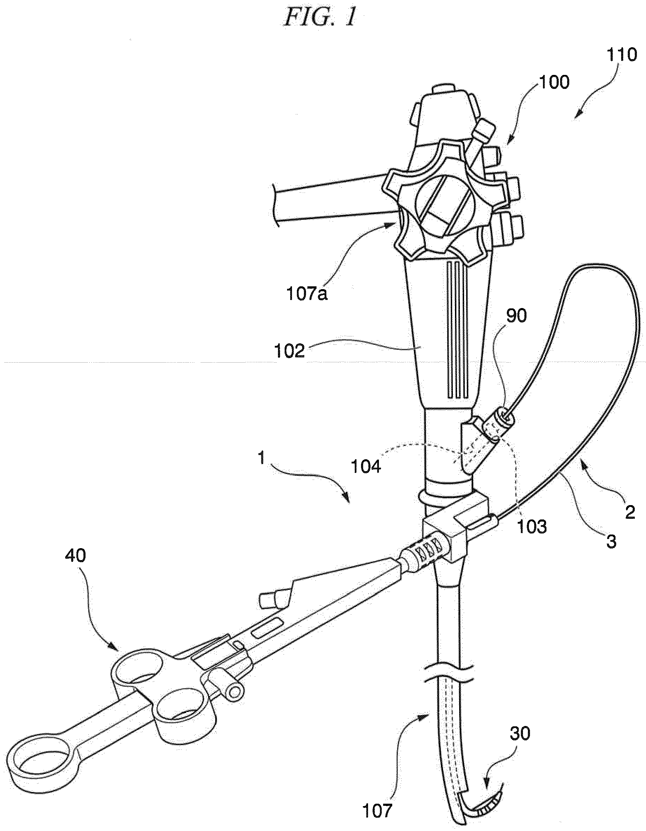

A first embodiment of the present invention will be described. FIG. 1 is an overall view of an incision system 110 which includes a treatment tool 1 for an endoscope according to the present embodiment.

As shown in FIG. 1, the treatment tool 1 for an endoscope according to the present embodiment is a medical instrument which is used along with an endoscope apparatus 100 in order to incise a biological tissue in the body. The treatment tool 1 for an endoscope configures an incision system 110 (endoscope treatment system) in a state of being combined with the endoscope apparatus 100. The endoscope apparatus 100 according to the present embodiment includes a bendable portion 107 (refer to FIG. 1). The bendable portion 107 is bent by operating a bending operation section 107a.

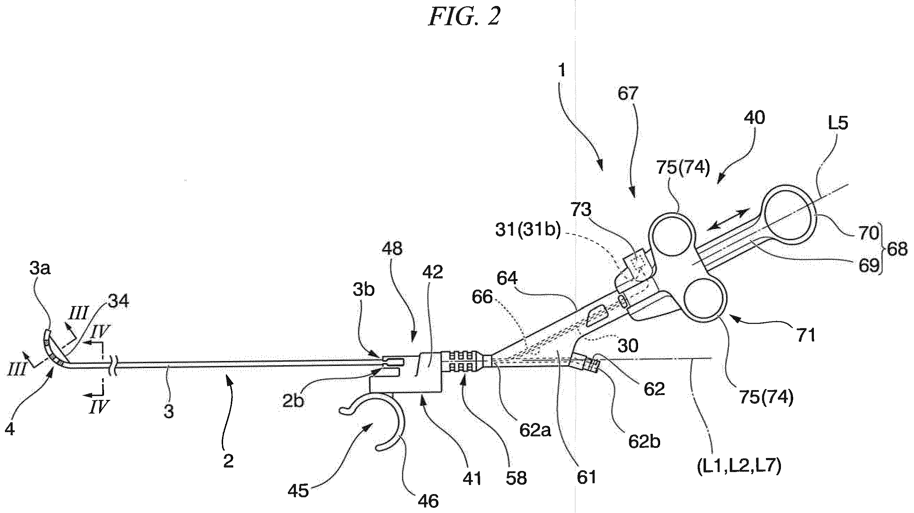

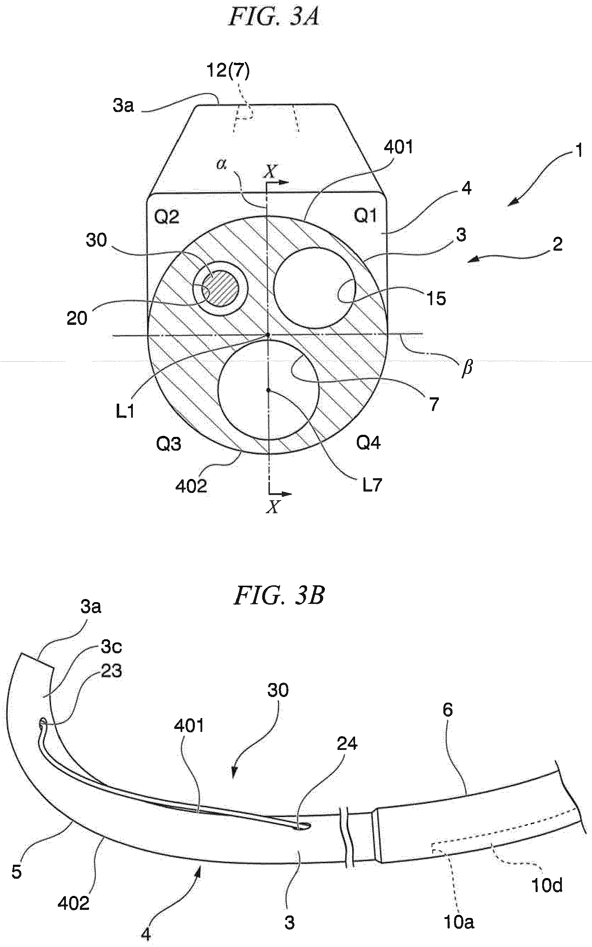

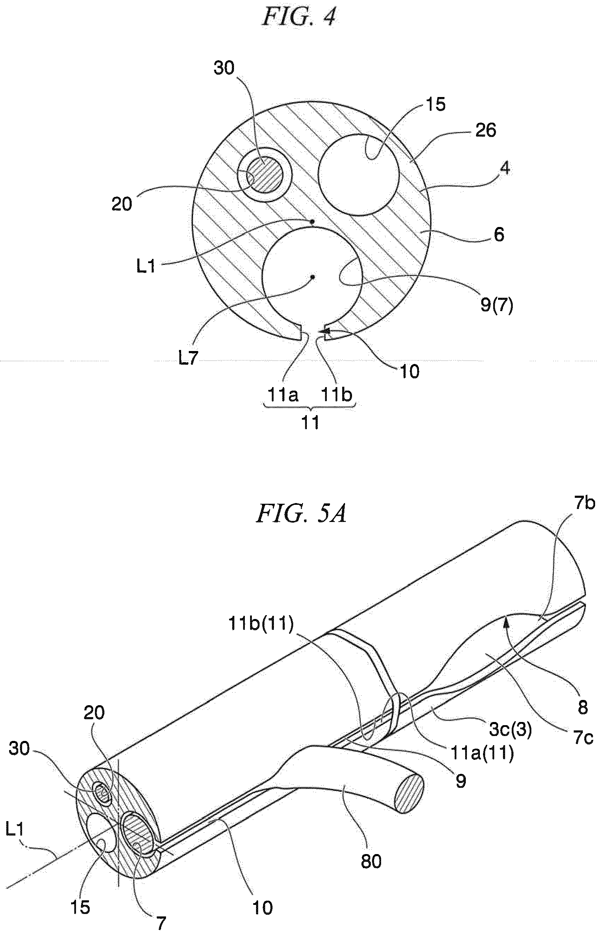

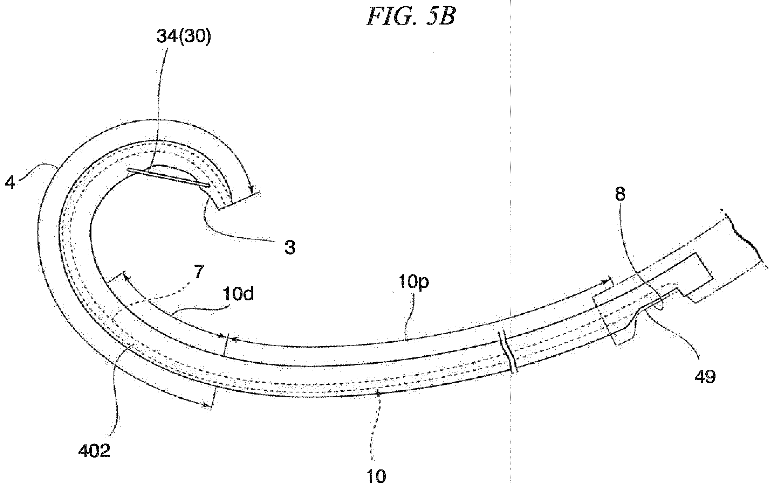

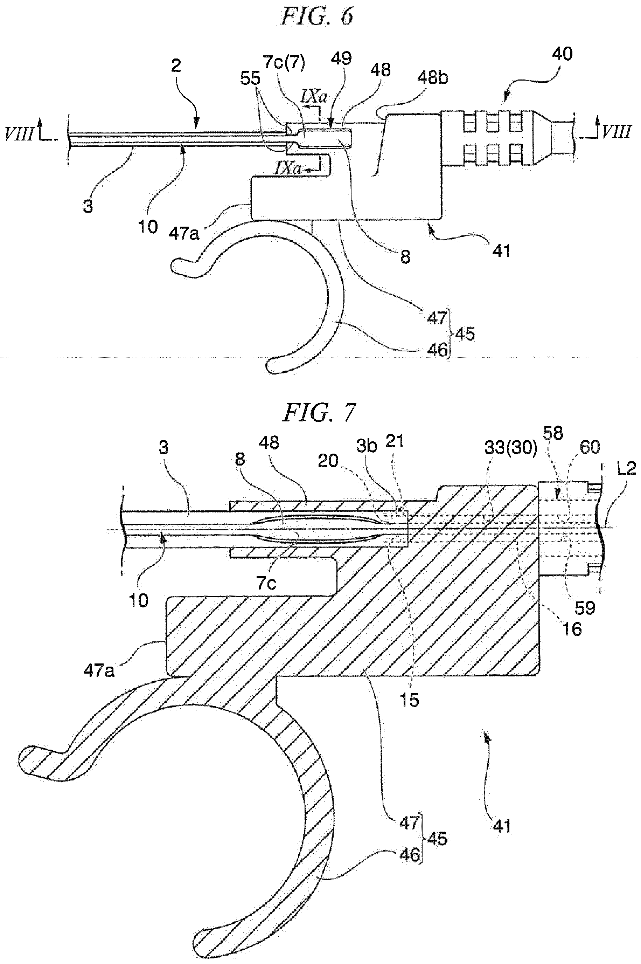

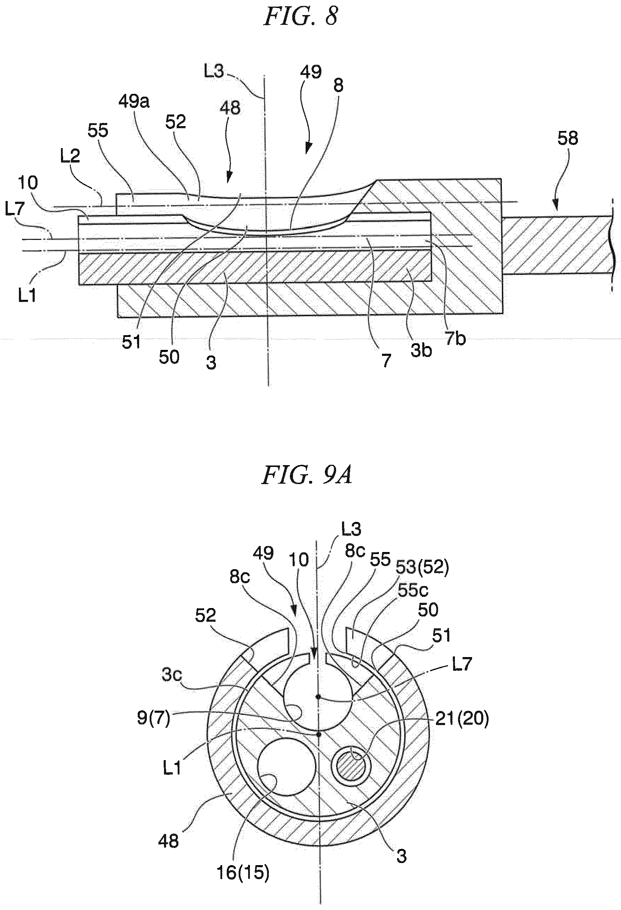

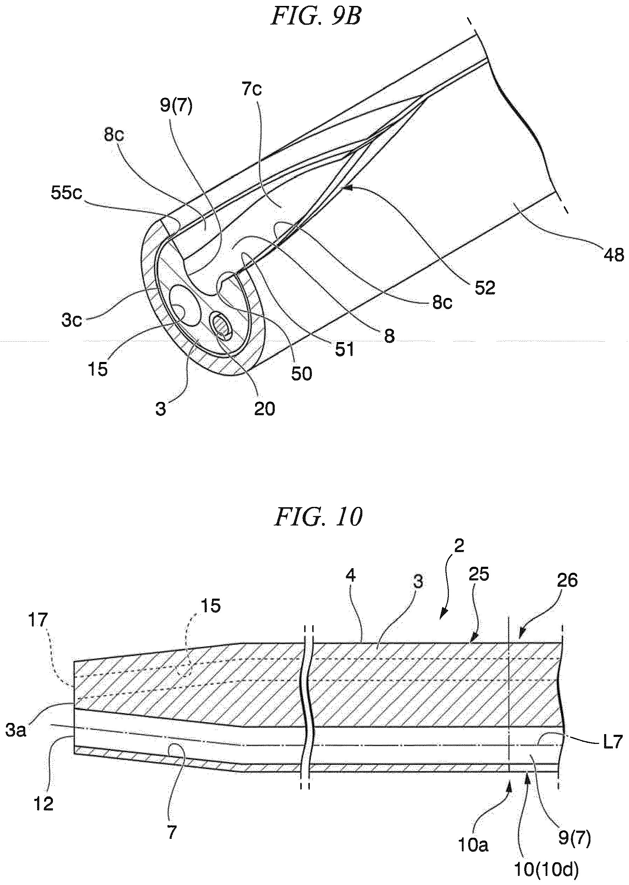

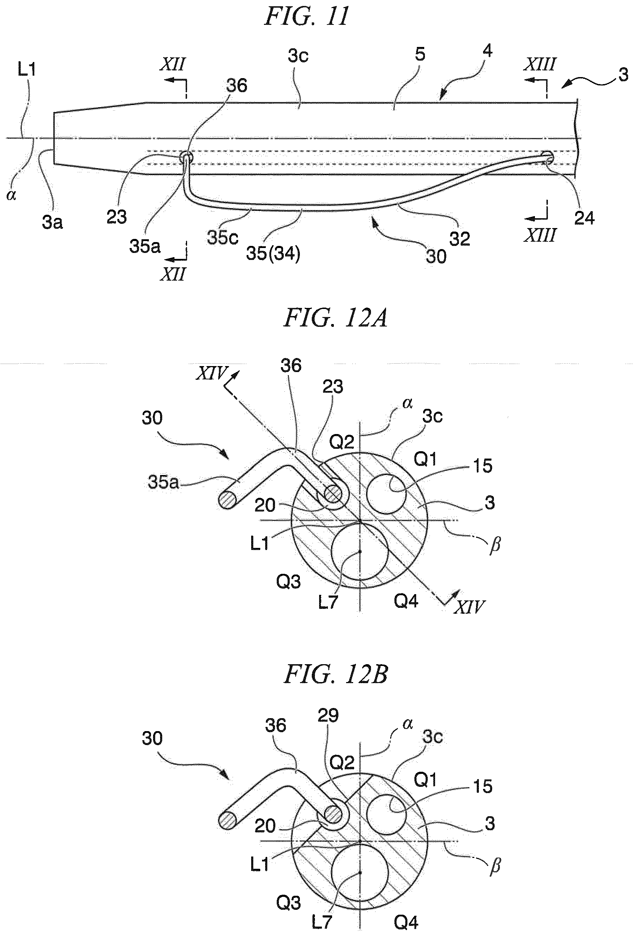

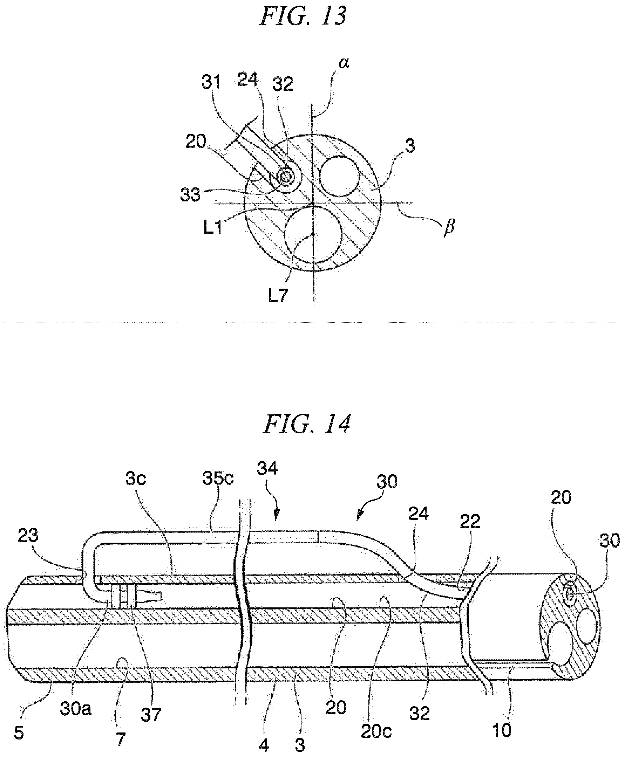

FIG. 2 is a plan view of the treatment tool 1 for an endoscope. FIG. 3A is a sectional view taken along line III-III of FIG. 2. FIG. 3B is a plan view showing a distal end portion of a sheath 3 of the treatment tool 1 for an endoscope. FIG. 4 is a sectional view taken along line IV-IV of FIG. 2. FIG. 5A is a perspective view showing a portion of the sheath 3 in the treatment tool 1 for an endoscope. FIG. 5B is a plan view showing the sheath 3. FIG. 6 is a plan view showing a portion of an operation portion 40 of the treatment tool 1 for an endoscope. FIG. 7 is a partial sectional view showing the sheath 3 and a distal configuration portion 41 of the operation portion 40 in the treatment tool 1 for an endoscope. FIG. 8 is a partial sectional view showing a first port 49 portion of the treatment tool 1 for an endoscope, and is a view when viewed from a direction of line VIII-VIII shown in FIG. 6. FIG. 9A is a sectional view taken along line IX-IX of FIG. 6. FIG. 9B is a perspective view showing the first port 49 portion of the treatment tool 1 for an endoscope. FIG. 10 is a sectional view showing the distal end portion of the sheath 3 on a first virtual plane α. FIG. 11 is a view when the distal portion of the sheath 3 is viewed from a direction perpendicular to a second virtual plane β. FIG. 12A is a sectional view taken along line XII-XII of FIG. 11. FIG. 13 is a sectional view taken along line XIII-XIII of FIG. 11. FIG. 14 is a view showing the distal portion of the sheath 3, and includes a partial sectional view of the sheath 3 when viewed from line XIV-XIV shown in FIG. 12A.

The treatment tool 1 for an endoscope includes an insertion portion 2 and an operation portion 40. The insertion portion 2 is an elongated member which is inserted into a treatment tool channel 104 of the endoscope apparatus 100. The insertion portion 2 includes the sheath 3 and a knife wire 30. As shown in FIGS. 1 and 2, the sheath 3 is an elongated member which has a center axis L1 along a longitudinal axis and has flexibility. In the present embodiment, the sheath 3 is formed of a resin.

Hereinafter, the operation portion 40 side of the treatment tool 1 for an endoscope is referred to as a proximal side, and a side on which the insertion portion 2 is provided and which is inserted into the body is referred to as a distal side.

As shown in FIG. 3B, the sheath 3 has a pre-curved portion 4 in a predetermined region including a distal end 3a of the sheath 3. A bending habit is applied to the pre-curved portion 4 so as to be curved in a shape which is curved in a predetermined direction, and the pre-curved portion 4 has a restoring force which restores the pre-curved portion 4 so as to be a predetermined curved shape. As shown in FIG. 3A, the center axis L1 of the sheath 3 exists in one predetermined plane (hereinafter, referred to as a "first virtual plane α") in the pre-curved portion 4. That is, the pre-curved portion 4 has a restoring force so as to follow the curved shape in which the sheath 3 is curved along the first virtual plane α.

A drawing portion 5 is provided at the distal portion side of the pre-curved portion 4, and a copying-deformation portion 6 is provided at the proximal portion side of the pre-curved portion 4. Preferably, an outer diameter of the drawing portion 5 has a diameter which is slightly smaller than outer diameters of the proximal end side and the copying-deformation portion 6 of the sheath 3.

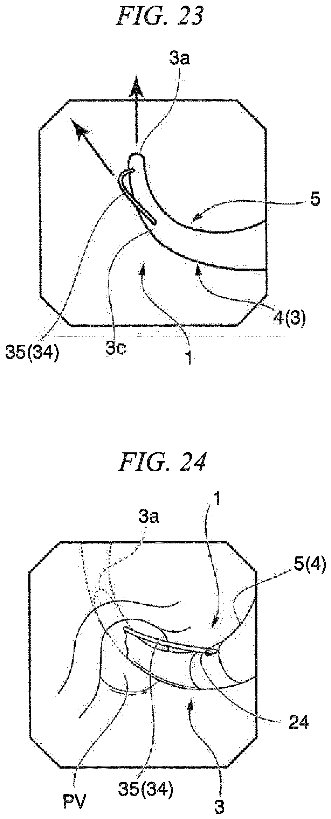

At least a portion of the drawing portion 5 is inserted into a duodenal papilla PV (refer to FIG. 23) of a patient who is an object to be treated. As shown in FIG. 3B, a first distal communication hole 23 and a second distal communication hole 24 described below are provided in the drawing portion 5.

The copying-deformation portion 6 is included in a distal portion of a soft region 26 described below in the present embodiment. In the present embodiment, a distal slit portion 10d described below is not formed in the drawing portion 5, and is formed in the copying-deformation portion 6.

As shown in FIG. 3A, the configuration of the sheath 3 is described using an orthogonal coordinate system (hereinafter, referred to as a "virtual coordinate system") in which the center axis L1 is an origin, the first virtual plane α is a vertical axis, and a plane (hereinafter, referred to as a "second virtual plane β") orthogonal to the first virtual plane α on the center axis L1 of the sheath 3 is a horizontal axis when a cross section orthogonal to the center axis L1 of the sheath 3 is viewed along the center axis L1 of the sheath 3 from the proximal end 3b of the sheath 3 toward the distal end 3a. In the vertical axis of the virtual coordinate system, the curved direction of the pre-curved portion 4 is referred to as an upper side.

As shown in FIGS. 3A and 5A, a first lumen 7, a second lumen 15, and a third lumen (knife wire lumen) 20 are formed inside the sheath 3. The first lumen 7, the second lumen 15, and the third lumen 20 are formed to extend so as to be parallel with one another in a longitudinal direction of the sheath 3. Each lumen has a center axis which extends to be approximately parallel with the center axis L1 of the sheath 3.

The first lumen 7 is a passage portion which has an inner diameter portion through which a guide wire 80 can move forward and backward. That is, the first lumen 7 is a lumen in which the guide wire 80 is held in the inner portion. A center axis L7 of the first lumen 7 is positioned at the first virtual plane α, and the first lumen 7 is positioned below the center axis L1 of the sheath 3, that is, is positioned on a third quadrant Q3 and a fourth quadrant Q4 of the virtual coordinate system. Specifically, the first virtual plane α crosses the internal space of the first lumen 7. In addition, the predetermined first virtual plane α includes the center axis L7 of the first lumen 7.

Moreover, the first lumen 7 is positioned at a position spaced from the third lumen 20 in a circumferential direction. In addition, in the present embodiment, the case where the first lumen 7 is used as the passage through which the guide wire 80 moves is exemplified. However, the present invention is not limited to the guide wire 80, and the first lumen 7 may be used as a passage through which other treatment tools move.

As shown in FIGS. 5A and 10, the first lumen 7 includes an outlet portion 12 which is open to the distal end 3a, a guide wire accommodation portion 9, a slit portion 10, and an inlet portion 8 which is open to the proximal end side. The guide wire accommodation portion 9 is a region in which a slit portion 10 is formed in the first lumen 7 and which communicates with an outer circumferential surface 3c of the sheath 3.

As shown in FIGS. 4 and 5A, the slit portion 10 has a shape in which a resin member configuring the sheath 3 is cut out in the center axis L1 direction of the sheath 3. Preferably, the slit portion 10 is an elongated notch which is open to the outer circumferential surface 3c of the sheath 3 such that the first lumen 7 communicates with the outside of the sheath 3, and which is formed so as to extend in the center axis L1 direction of the sheath 3. As shown in FIG. 5B, the slit portion 10 includes a proximal slit portion 10p which is formed in a region closer to the proximal side of the pre-curved portion 4 relative to the proximal end thereof. In this way, since the proximal slit portion 10 is formed, a cross-sectional torsion moment decreases, and twisting is more easily performed in the region of the proximal side of the pre-curved portion 4 relative to the proximal end of the pre-curved portion 4.

Preferably, the proximal slit portion 10p extends from the proximal end of the pre-curved portion 4 to the inlet portion 8, and communicates with the opening of the inlet portion 8. The inlet portion 8 is open so as to communicate with the outer circumferential surface 3c of the sheath 3 from the inner portion of the guide wire accommodation portion 9 at the proximal end side of the sheath 3, and the guide wire 80 can be introduced into the inlet portion 8.

The guide wire accommodation portion 9 is formed along the longitudinal axis of the sheath 3 at a position spaced from the third lumen 20 in the circumferential direction around the center axis of the sheath 3.

The slit portion 10 may further include the distal slit portion 10d. As shown in FIG. 5B, the distal slit portion 10d extends from the proximal end of the pre-curved portion 4 to the intermediate portion of the pre-curved portion 4 toward the distal side thereof, and may be formed along the center axis of the sheath 3 such that the guide wire accommodation portion 9 communicates with an outer circumferential surface 402 in an outward side of the curved shape of the pre-curved portion 4. Specifically, the distal slit portion 10d is formed along the center axis L1 of the sheath 3 at a position at which the outer circumferential surface of the copying-deformation portion 6 and the first virtual plane α intersect each other. The position of a distal end 10a of the distal slit portion 10d is positioned at a position closer to the proximal side relative to the proximal end of the drawing portion 5, and is positioned at an arbitrary position (the intermediate portion of the copying-deformation portion 6) at the copying-deformation portion 6 or the distal end of the copying-deformation portion 6. The proximal end of the distal slit portion 10d is positioned at the proximal end of the pre-curved portion 4. Preferably, the distal slit portion 10d communicates with the proximal slit portion 10p. If external force is applied to the copying-deformation portion 6 in which the distal slit portion 10d is formed, a contour shape of the copying-deformation portion 6 in the cross section orthogonal to the center axis L1 of the sheath 3 has rigidity by which the copying-deformation portion 6 is easily deformed in an elliptical shape.

A region from the distal end 3 a of the sheath 3 to the distal end 10a of the distal slit portion 10d is a high-rigidity region 25 which has relatively high torsional rigidity in the sheath 3. Meanwhile, a region from the distal end of the proximal slit portion 10p to the distal end of the notch portion 55 (refer to FIG. 6) is set to a soft region 26 which has at least lower torsional rigidity relative to the high-rigidity region 25. A restoring force of the high-rigidity region 25 when it is restored in a predetermined curved shape in the pre-curved portion 4 is larger than the restoring force of the soft region 26. Accordingly, the high-rigidity region 25 easily follows the bending shape of the bendable portion 107 in the treatment tool channel 104 which is bent by the bendable portion 107 of the endoscope apparatus 100, and when the high-rigidity region 25 protrudes from the distal end 104a of the treatment tool channel 104 of the endoscope apparatus 100, the high-rigidity region 25 is restored to a curved shape suitable for incision of a target portion to be treated.

As shown in FIG. 4, the slit portion 10 has a pair of flap portions 11 (first flap 11 a and second flap 11b) which is disposed so as to be spaced from each other such that an opening width of the slit portion 10 is smaller than the diameter of the guide wire 80. The flap portions 11 are a pair of elastic portions which covers the guide wire accommodation portion 9 by a resin member configuring the sheath 3. The flap portions 11 are deformed until a gap is generated, which has a size by which the guide wire 80 can pass through by force of an operator when the guide wire 80 is detached from the guide wire accommodation portion 9 through the slit portion 10.

As shown in FIG. 5A, the inlet portion 8 is a portion which is open to the outer circumferential surface 3c of the sheath 3 so as to have the same size as the diameter of the guide wire 80 or to have a larger size than the diameter of the guide wire 80 in the vicinity of a proximal end 7b of the first lumen 7. In other words, the inlet portion 8 is an opening portion in which an inner surface 7c of the first lumen 7 is exposed to the outside in a state where the flap portions 11 are not provided, and which has a wider width than that of the slit portion 10.

The length of inlet portion 8 in the center axis L1 direction of the sheath 3 is larger than the inner diameter of the guide wire accommodation portion 9 (first lumen 7) in the first lumen 7. That is, the inlet portion 8 has a long hole shape which is long in the center axis L1 direction of the sheath 3. In addition, the shape of the inlet portion 8 may have a rectangular shape. If the shape of the inlet portion 8 is rectangular, the inlet portion 8 is easily processed. Right and left ends 8c (both ends in the circumferential direction) of the inlet portion 8 in the circumferential direction of the sheath 3 may have a taper shape (refer to FIG. 9A) in which an opening area of the inlet portion 8 gradually increases from the guide wire accommodation portion 9 toward the outer circumferential surface 3c of the sheath 3.

As shown in FIG. 4, the guide wire accommodation portion 9 has a circular contour except for a boundary between the guide wire accommodation portion 9 and the slit portion 10 in the cross section orthogonal to the center axis L1 of the sheath 3. That is, the guide wire accommodation portion 9 has an approximately C-shaped contour shape in the cross section orthogonal to the center axis L1 of the sheath 3. The guide wire accommodation portion 9 has clearance in a state where the guide wire 80 is inserted into the guide wire accommodation portion 9 such that the guide wire 80 can move forward and backward, and the inner diameter of the guide wire accommodation portion 9 is larger than the diameter of the guide wire 80 by the dimensions of the clearance.

As shown in FIG. 3A, the first lumen 7 has a continuous circumferential contour when viewed from a cross section orthogonal to the center axis L1 of the sheath 3 in the region between the distal end 10a of the distal slit portion 10d and the distal end 3a of the sheath 3, wherein the circumferential contour functions as a distal region which regulates the movement of the guide wire 80 in the radial direction of the first lumen 7. In addition, as shown in FIG. 10, the outlet portion 12 which is open to communicate with the guide wire accommodation portion 9 is provided at the distal end 3a of the sheath 3, and the guide wire 80 can protrude from the outlet portion 12.

For example, the second lumen 15 is a liquid-feeding lumen for feeding liquid such as a contrast agent from the proximal end 3b (refer to FIG. 2) of the sheath 3 to the distal end 3a (FIGS. 2 and 3A) of the sheath 3. In addition, the second lumen 15 can be used as a liquid-discharging lumen for removing liquid in the body.

The second lumen 15 in the pre-curved portion 4 is positioned at a first quadrant Q1 in the virtual coordinate system.

A second port 62 described below is provided at the proximal end of the second lumen 15. The second port 62 has an opening through which liquid is introduced. The distal end of the second lumen 15 has an opening (distal discharging port) 17 through which the liquid introduced from the second port 62 is discharged.

As shown FIGS. 3A, 4, and 5A, the third lumen 20 is a lumen into which the knife wire 30 described below is inserted. The third lumen 20 is set such that the knife wire 30 can move forward and backward in the third lumen 20. That is, the third lumen 20 has clearance in a state where the knife wire 30 is inserted into the third lumen 20, and the inner diameter of the third lumen 20 is larger than the diameter of the knife wire 30 by the dimensions of the clearance. The third lumen 20 in the pre-curved portion 4 is positioned at a second quadrant Q2 in the virtual coordinate system.

As shown in FIG. 3A, in the state where the pre-curved portion 4 is restored to the curved shape, the second lumen 15 and the third lumen 20 are positioned in a region of an inner surface 401 side of the curved shape, and the first lumen 7 is positioned in a region of an outer surface 402 side of the curved shape. That is, as shown in FIG. 3A, in the vicinity of the distal end 3 a of the sheath 3, when viewed from the viewpoint of a dial plate of a timepiece in which the upper side (the upper side of first virtual plane α) of the vertical axis in the virtual coordinate system of the cross section orthogonal to the center axis L1 of the sheath 3 is set to twelve o'clock, the third lumen 20 in the pre-curved portion 4 is positioned within a range between nine o'clock and twelve o'clock.

The third lumen 20 includes a knife wire accommodation portion 22 (refer to FIG. 14), a first distal communication hole 23, and a second distal communication hole 24.

As shown in FIG. 3A, the first virtual plane α crosses a wall portion 3d which is positioned between the second lumen 15 and the third lumen 20 in the sheath 3. In the present embodiment, the wall portion 3d is positioned on the first virtual plane α, and the second lumen 15 and the third lumen 20 are positioned on both sides in the state where the first virtual plane α is interposed therebetween.

The knife wire accommodation portion 22 covers the entire outer circumference of the knife wire 30 so as to maintain the knife wire 30 in an electrically insulated state.

As shown in FIGS. 11 and 14, the first distal communication hole 23 is open to the outer circumferential surface 3c of the sheath 3, and communicates with the third lumen 20. The first distal communication hole 23 is positioned at a second quadrant Q2 in the virtual coordinate system. That is, when viewed from the viewpoint of a dial plate of a timepiece in which the upper side (the upper side of first virtual plane α) of the vertical axis in the virtual coordinate system of the cross section orthogonal to the center axis L1 of the sheath 3 is set to twelve o'clock, the first distal communication hole 23 is positioned within a range between nine o'clock and twelve o'clock. Specifically, the first distal communication hole 23 communicates with the inner surface 401 (the outer circumferential surface positioned at the inward side of the curved shape) of the curved shape and the third lumen 20 in the pre-curved portion 4. In addition, the first distal communication hole 23 is formed so as to be open at the position spaced from the first virtual plane α on the inner surface 401 side of the curved shape in the distal portion of the pre-curved portion 4. In addition, the first distal communication hole 23 is formed so as to be open in the direction spaced from the position of the third lumen 20 toward the outside in the radial direction with respect to the center axis of the sheath 3.

The second distal communication hole 24 is positioned at a position spaced from the first distal communication hole 23 so as to be closer to the proximal side relative to the first distal communication hole 23. The second distal communication hole 24 is positioned at the second quadrant Q2 in the virtual coordinate system. That is, when viewed from the viewpoint of a dial plate of a timepiece in which the upper side (the upper side of first virtual plane α) of the vertical axis in the virtual coordinate system of the cross section orthogonal to the center axis L1 of the sheath 3 is set to twelve o'clock, similarly to the first communication hole 23, the second distal communication hole 24 is positioned within a range between nine o'clock and twelve o'clock. Specifically, similarly to the first distal communication hole 23, the second distal communication hole 24 communicates with the curved inner surface 401 (the outer circumferential surface positioned at the inward side of the curved shape) and the third lumen 20 in the pre-curved portion 4. In addition, the second distal communication hole 24 is formed so as to be open at the position spaced from the first virtual plane α on the inner surface 401 side of the curved shape in the distal portion of the pre-curved portion 4. Moreover, the second distal communication hole 24 is formed so as to be open in the direction spaced from the position of the third lumen 20 toward the outside in the radial direction with respect to the center axis of the sheath 3. In addition, preferably, the first distal communication hole 23 and the second distal communication hole 24 are disposed in the region in which the drawing portion 5 exists. In addition, in the cross section orthogonal to the center axis L1 of the sheath 3, preferably, the positions of the first distal communication hole 23 and the second distal communication hole 24 in the circumferential direction with the center axis L1 of the sheath 3 as a center coincide with each other. However, the positions of the first distal communication hole 23 and the second distal communication hole 24 in the circumferential direction of the sheath 3 do not necessarily need to coincide with each other. The first distal communication hole 23 and the second distal communication hole 24 can exert the functions if the holes 23 and 24 are provided so as to communicate with the inner surface 401 of the curved shape and the third lumen 20 in the pre-curved portion 4, and are open in the direction spaced from the center axis L1 of the sheath 3 toward the outside in the radial direction from the position of the third lumen 20 at the position spaced from the first virtual plane α on the inner surface 401 side of the curved shape in the distal portion of the pre-curved portion 4.

As shown in FIG. 11, the knife wire 30 includes a cutting portion 34 which incises a target portion to be treated. The cutting portion 34 protrudes from the first distal communication hole 23 and the second distal communication hole 24, extends between the first distal communication hole 23 and the second distal communication hole 24, and is provided so as to incise tissues. Preferably, as shown in FIG. 13, the knife wire 30 includes a core wire 31 having conductivity, and an insulating film 32 which covers the core wire 31. In addition, the knife wire 30 includes a distal fixing member (fixing portion) 37.

For example, the insulating film 32 is formed by coating or covering resins such as polytetrafluoroethylene (PTFE), tetrafluoroethylene-hexafluoropropylene resin (FEP), polyethylene, polyolefin, polyamide, vinyl chloride, latex, natural rubber, polysulfone, polyphenylsulfon, polyetherimide, POM, PEEK, polycarbonate, or ABS, or combined resin materials thereof on the outer surface of the core wire 31.

As shown in FIG. 11, the cutting portion 34 is a portion in which the core wire 31 is not covered on the insulating film 32 (refer to FIG. 13) over the entire length of the knife wire 30 and which is disposed outside the sheath 3. The cutting portion 34 can incise biological tissues by energizing a high-frequency current supplied to the core wire 31 via a connector 73. Preferably, the cutting portion 34 includes a bending portion 36 described below in addition to a curved knife portion 35.

As shown in FIGS. 13 and 14, the proximal end of the knife wire 30 is fixed to a slider portion 71 of a handle portion 67 in the operation portion 40 (refer to FIG. 2). The knife wire 30 is inserted into the third lumen 20, and is fixed to the third lumen 20 by the distal fixing member 37. In addition, the core wire 31 of the knife wire 30 may be covered with the insulating film 32 from the proximal end of the knife wire 30 to the proximal end of the cutting portion 34.

As shown in FIGS. 11, 12A, and 13, the curved knife portion 35 is disposed at the inward side of the curve of the pre-curved portion 4 at the second quadrant Q2 in the virtual coordinate system in the region between the first distal communication hole 23 and the second distal communication hole 24. As shown in FIGS. 11, 12A, 13, and 14, the bending portion 36 is formed at the distal end 35a of the curved knife portion 35. The maximum portion 35c is formed at the curved knife portion 35 between the first distal communication hole 23 and the second distal communication hole 24. The maximum portion 35c of the curved knife portion 35 has a curved shape which is disposed across a portion of the pre-curved portion 4 between the first distal communication hole 23 and the second distal communication hole 24 at a position spaced from the first virtual plane α. The maximum portion 35c of the curved knife portion 35 is positioned at a position close to the first distal communication hole 23 from the center between the first distal communication hole 23 and the second distal communication hole 24 in the center axis L1 direction of the sheath 3.

Moreover, instead of the above-described configuration, as shown in FIG. 12B, the first distal communication hole 23 may be formed so as to be notched from the outer circumferential surface 3c of the pre-curved portion 4 at the position of the inner surface 401 of the curved shape in the pre-curved portion 4 (sheath 3). Specifically, the first distal communication hole 23 may be a notch portion 29 which is notched so as to communicate with the third lumen 20 from the outer circumferential surface 3c of the pre-curved portion 4 at the position of the second quadrant (between nine o'clock and twelve o'clock). Similarly to the first distal communication hole 23, the second distal communication hole 24 may also be formed so as to be notched from the outer circumferential surface 3c of the pre-curved portion 4 at the position of the inner surface 401 of the curved shape in the pre-curved portion 4 (sheath 3). Specifically, the second distal communication hole 24 may be a notch portion 29 which is notched so as to communicate with the third lumen 20 from the outer circumferential surface 3c of the pre-curved portion 4 at the position of the second quadrant (between nine o'clock and twelve o'clock).

As shown in FIG. 12A, the bending portion 36 is bent in the direction away from the first virtual plane α.

The curved knife portion 35 is curved so as to reach the second distal communication hole 24 from the first distal communication hole 23 on a virtual plane which is approximately parallel with a tangential plane which intersects a first virtual plane α within the range of the second quadrant Q2 and comes into contact with the outer circumferential surface 3 c of the sheath 3 in the virtual coordinate system defined by the first virtual plane α and the second virtual plane β, and the curved knife portion 35 is curved such that the maximum portion 35c is spaced farthest from the first virtual plane α at the curved knife portion 35.

For example, the maximum portion 35c may be curved so as to be inclined with respect to the first virtual plane α and the second virtual plane β such that the maximum portion 35c approaches the second virtual plane β as the distance between the maximum portion 35c and the bending portion 36 increases. In addition, the maximum portion 35c may be curved so as to be inclined with respect to the first virtual plane α and the second virtual plane β such that the maximum portion 35c is away from the second virtual plane P as the distance between the maximum portion 35c and the bending portion 36 increases. The maximum portion 35c may extend so as to be approximately parallel with the second virtual plane β.

As shown in FIGS. 11 and 12A, when the distal end 35a of the curved knife portion 35 is viewed from the direction orthogonal to the center axis L1 of the sheath 3, it is preferable that the direction of the distal end 35a of the curved knife portion 35 is along the plane orthogonal to the center axis L1 of sheath 3. However, the direction of the distal end 35a may be a direction toward the proximal side of the sheath 3 slightly away from the plane orthogonal to the center axis L1 of the sheath 3 (longitudinal axis of the sheath 3).

The bending portion 36 may have a shape in which the knife wire 30 protruding from the first distal communication hole 23 is bent with respect to the protrusion direction in the direction approximately parallel with a tangential line to the outer circumferential surface 3c of the sheath 3 at the second quadrant Q2 in the virtual coordinate system defined by the first virtual plane α and the second virtual plane β. Specifically, the cutting portion 34 may have the bending portion which is bent in the direction of enlarging a space from the first virtual plane α against the direction in which the first distal communication hole 23 is open. In the knife wire 30, the bending portion 36 is a portion in which the core wire 31 is bent such that the core wire 31 extending from the curved knife portion 35 toward the distal end 30a of the knife wire 30 is bent toward the first distal communication hole 23. The bending portion 36 may be covered with the insulating film 32.

The distal fixing member 37 is fixed to the distal end 30a of the knife wire 30, and is fixed to the inner portion of the third lumen 20. That is, in the state where the distal fixing member 37 is inserted into the pre-curved portion 4, the knife wire 30 and the pre-curved portion 4 are fixed to each other by the distal fixing member 37. In addition, the distal fixing member 37 is connected to an inner circumferential surface 20c of the third lumen 20 in the pre-curved portion 4 by friction, bonding, or other connection methods. Since the distal fixing member 37 is fixed to the inner portion of the third lumen 20 (pre-curved portion 4), the distal portion of the knife wire 30 is not extracted from the first distal communication hole 23.

Since the knife wire 30 is fixed to the pre-curved portion 4, if the knife wire 30 is pulled toward the proximal end side, the drawing portion 5 in the vicinity in which the first distal communication hole 23 is provided is pulled toward the proximal end side, and the drawing portion 5 is curved so as to be larger than a curved shape applied in advance. That is, the knife wire 30 has a function of curving the drawing portion 5 to be equal to or more than a curved angle applied in advance, in addition to the function of incising the target portion to be treated.

The operation portion 40 shown in FIG. 2 is a portion which is held by an operator, and is disposed at the proximal end 2b (proximal end 3b of sheath 3) of the insertion portion 2. Various operations for operating the treatment tool 1 for an endoscope are input to the operation portion 40.

The operation portion 40 includes a distal configuration portion 41, a flexible connection portion 58, a proximal configuration portion 61, and a handle portion 67.

As shown in FIG. 6, the distal configuration portion 41 is a member which is disposed at the most distal side in the operation portion 40. The distal configuration portion 41 includes a connection portion 45 to the endoscope apparatus 100, and a connection portion 48 to the sheath 3.

The connection portion 45 has a hook 46. The hook 46 is a locking portion which can be locked to the holding portion 102 which is provided in the endoscope apparatus 100.

The hook 46 is an elastic member which is formed in a C shape so as to surround a portion of the outer circumferential surface of the holding portion 102 which is provided in the endoscope apparatus 100. The hook 46 can press the outer surface of the holding portion 102 of the endoscope apparatus 100 by a restoring force by which the hook 46 is restored to a C shape. As a result, the hook 46 can engage with the holding portion 102 of the endoscope apparatus 100.

The connection portion 48 is a tubular portion which is formed in an approximately tubular shape having an inner diameter portion into which the proximal end 3b of the sheath 3 and the vicinity thereof can be inserted, and includes the first port 49. In addition, the operation portion 40 is fixed to the sheath 3 by the connection portion 48 in the proximal end 3b of the sheath 3 and in the vicinity thereof such that the opening directions of the first port 49 and the inlet portion 8 coincide with each other. As a result, the sheath 3 is not rotated around the longitudinal axis and is not extracted from the tubular portion. As a method for fixing the operation portion 40 and the sheath 3 to each other, a known fixing method can be appropriately adopted.

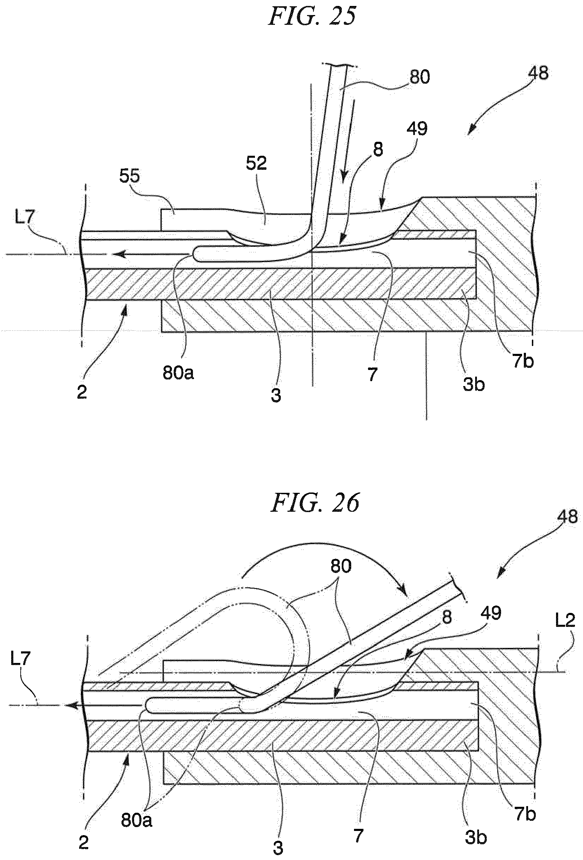

As shown in FIGS. 6, 8, and 9B, the first port 49 is a port which becomes an inlet through which the guide wire 80 (refer to FIG. 25) is introduced into the first lumen 7. The first port 49 includes an inner opening edge portion 50, an outer opening edge portion 51, a tapered portion 52, and a notch portion 55.

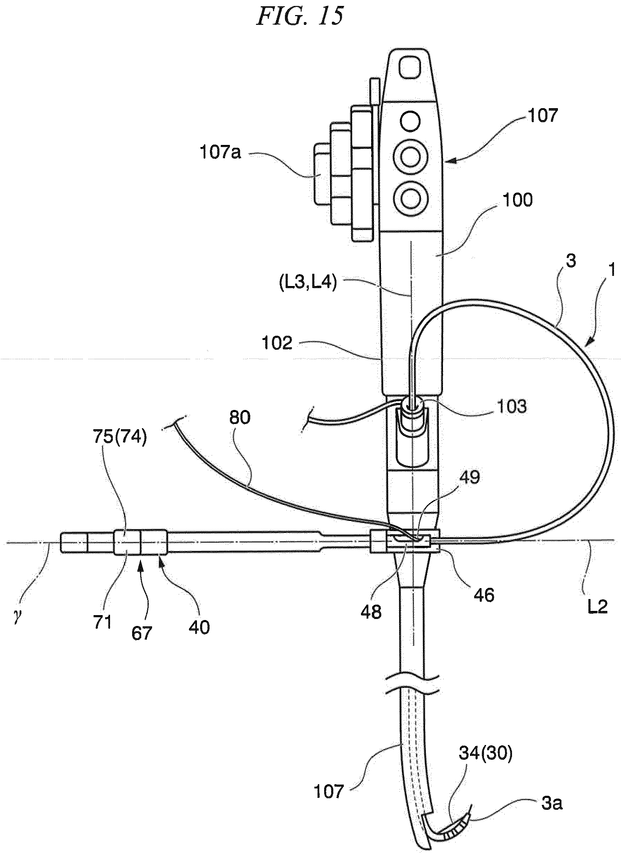

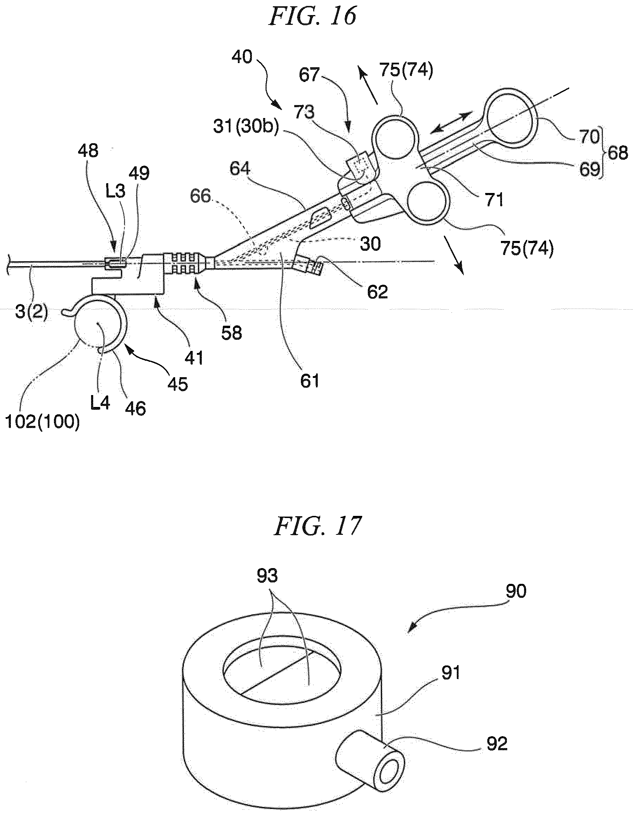

FIG. 15 is a view showing a positional relationship between the first port 49 and the operation portion 40 in a state where the treatment tool 1 for an endoscope is attached to the endoscope apparatus 100. FIG. 16 is a view showing a positional relationship between the hook 46 and the first port 49 in a state where the treatment tool 1 for an endoscope is attached to the endoscope apparatus 100.

As shown in FIG. 15, in a state where the operation portion 40 is locked to the holding portion 102 of the endoscope apparatus 100 by the hook (locking portion) 46, the positional relationship between the opening of the first port 49 and the hook 46 is determined such that the opening of the first port 49 faces the bending operation section 107a side of the endoscope apparatus 100. Here, the bending operation section 107a is an operation portion capable of actively operating the bendable portion 107 which is provided at the distal portion of the endoscope apparatus 100. In addition, the bendable 107 actively bends a treatment tool channel which is disposed in an endoscope insertion portion which is inserted into the body. In the first port 49, a through hole may be formed, which has an axis direction, which intersects a virtual plane penetrating along an axis approximately parallel with the longitudinal direction of the holding portion 102, as a penetrating direction (axis direction). The treatment tool 1 for an endoscope may be fixed to the endoscope apparatus 100 so as to extend in a direction approximately parallel with a direction in which a treatment tool channel port 103 of the endoscope apparatus 100 extends, and in this case, the axis direction (center axis L3 direction described below) in which the through hole of the first port 49 penetrates has a positional relationship which is twisted with respect to the longitudinal axis of the holding portion 102 of the endoscope apparatus 100.

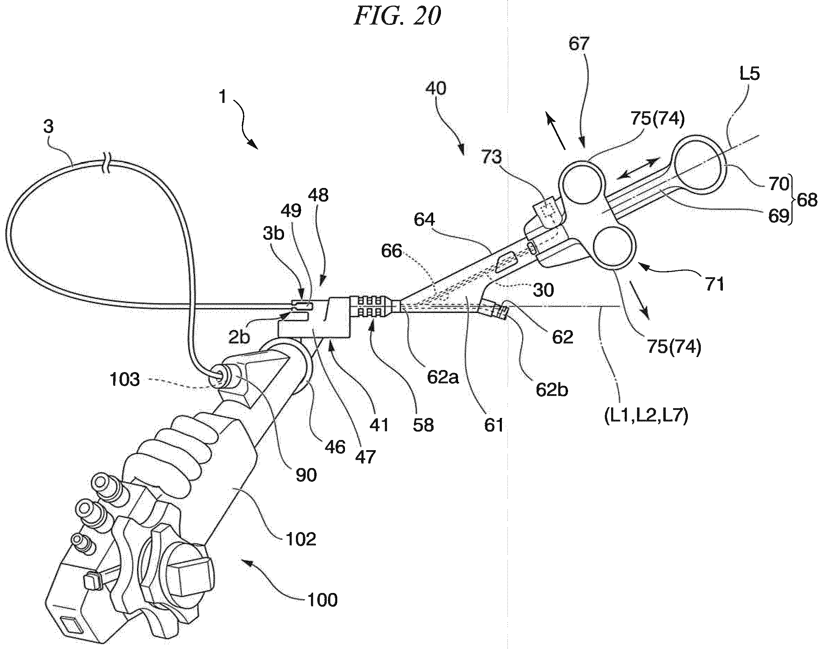

In the present embodiment, in the opening of the first port 49, a straight-line direction (refer to FIG. 8) (hereinafter, referred to as a "center axis L3 direction of the opening of the first portion 49") from the plane defined by the inner opening edge portion 50 toward the plane defined by the outer opening edge portion 51 is approximately parallel with a center axis L4 direction of a circle surrounding the holding portion 102 in the hook 46, as shown in FIG. 16. That is, in the cross section of the operation portion 40 of the treatment tool for an endoscope 1 shown in FIG. 9A, the center axis L3 direction of the opening of the first port 49 passing through a portion between the inner opening edge portions 50 and a portion between outer opening edge portions 51 through a center axis L7 of the first lumen 7 is approximately parallel with the center axis L4 direction of the circle surrounding the holding portion 102 in the hook 46. As a result, as shown in FIG. 9A, the opening of the first port 49 is formed so as to be open with the center axis L3 of the opening of the first port 49 orthogonal to the center axis L1 of the sheath 3 as the center, and the center axis L3 of the opening of the first port 49 is approximately parallel with the center axis L4 of the circle surrounding the holding portion 102 of the endoscope apparatus 100. Accordingly, in the state where the operation portion 40 is attached and locked to the endoscope apparatus 100 by the hook 46, the opening of the first port 49 faces the direction (refer to FIG. 20) which is visible by an operator in a general positional relationship in which the operator holds the holding portion 102 of the endoscope apparatus 100 so as to operate the endoscope apparatus 100.

As shown in FIGS. 8 and 9A, the notch portion 55 is a portion in which a notch is formed so as to have an approximately C shape when the connection portion 48 is viewed from the cross section orthogonal to the center axis L1 of the sheath 3. In addition, the notch portion 55 communicates with the opening of the first port 49. The notch portion 55 has a gap in which the diameter of the connection portion 48 is larger than the diameter of the guide wire 80 by a clearance through which the guide wire 80 can move such that the guide wire 80 can move from the inlet portion 8 to the slit portion 10. The gap in the notch portion 55 is provided along the slit portion 10 of the sheath 3. The notch portion 55 has an inner surface 55c which can come into contact with the outer circumferential surface 3c of the sheath 3, and holds the outer circumferential surface 3c of the sheath 3.

As shown in FIGS. 2 and 7, the flexible connection portion 58 is a member which connects the distal configuration portion 41 and the proximal configuration portion 61, and has flexibility. The flexible connection portion 58 buffers twisting generated between the distal configuration portion 41 and the proximal configuration portion 61. That is, in a case where the operation portion 40 and the endoscope apparatus 100 are connected to each other by the hook 46, the flexible connection portion 58 buffers the twisting which is generated between the distal configuration portion 41 and the proximal configuration portion 61 due to the operation of the operation portion 40 and the operation of the endoscope apparatus 100.

The flexible connection portion 58 includes a liquid-feeding communication passage 59 and a knife wire communication passage 60 inside the flexible connection portion 58. The liquid-feeding communication passage 59 communicates with the second lumen 15. The knife wire communication passage 60 communicates with the third lumen 20.

As shown in FIG. 2, the proximal configuration portion 61 includes a second port 62. The second port 62 is a port to which a syringe or the like in which liquid is accommodated is connected. For example, in a procedure in which a contrast agent is discharged from the distal end 3 a of the sheath 3 through the second lumen 15, a syringe which is filled with the contrast agent is connected to the second port 62. A proximal end 62b of the second port 62 has a connector structure which can be connected to the syringe having a Luer-lock structure. A distal end 62a of the second port 62 communicates with the liquid-feeding communication passage 59 (refer to FIG. 7) which is formed in the flexible connection portion 58.

The handle portion 67 shown in FIG. 2 includes a handle-fixing portion 64 and a knife wire passage 66. The handle-fixing portion 64 is provided so as to fix the handle portion 67 to the flexible connection portion 58 in a predetermined connection state. The knife wire passage 66 communicates with the knife wire communication passage 60 in the flexible connection portion 58, and is a passage into which the knife wire 30 is inserted so as to move forward and backward.

Inputs for operating the knife wire 30 are applied to the handle portion 67 by an operator. The handle portion 67 includes a shaft portion 68 and a slider portion 71. The shaft portion 68 is fixed to the handle-fixing portion 64 of the proximal configuration portion 61. The slider portion 71 is slidably connected along the longitudinal axis of the shaft portion 68.

The shaft portion 68 includes a rod-shaped portion 69 and a ring portion 70. The rod-shaped portion 69 extends so as to be coaxial with a center axis L5 of the handle-fixing portion 64 or so as to be linear along the center axis L5 of the handle-fixing portion 64. The ring portion 70 is formed at the proximal end of the rod-shaped portion 69. The ring portion 70 is an annular portion through which fingers of an operator can pass.

The slider portion 71 may include a connector 73 which can be connected to a high-frequency power supply device and a finger-hooking portion 74. Two rings 75 through which fingers of an operator can pass are formed at the finger-hooking portion 74. The proximal end of the knife wire 30 is electrically connected to the connector 73. Fingers of an operator pass through the two rings 75 and the ring portion 70, and thus, the finger-hooking portion 74 can be used to move the knife wire 30 forward and backward.

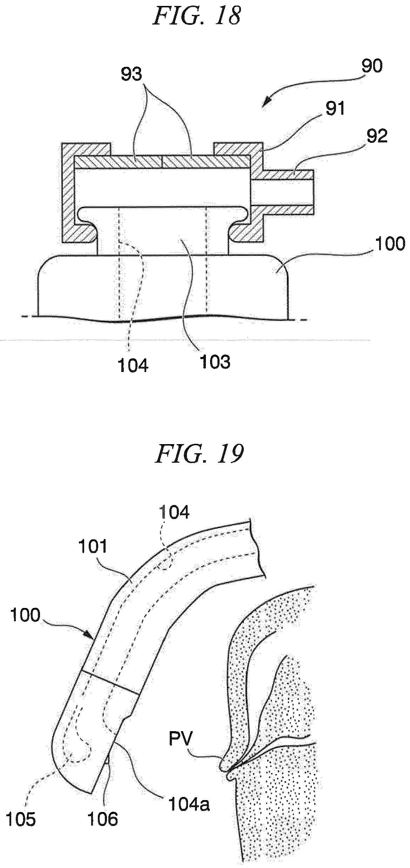

Next, a configuration of a treatment tool attachment-assisting instrument 90 will be described, which can be used in the procedure in which the treatment tool 1 for an endoscope according to the present embodiment is attached to the endoscope apparatus 100. FIG. 17 is a perspective view showing the treatment tool attachment-assisting instrument 90 which can be attached to the endoscope apparatus 100. FIG. 18 is a partial sectional view showing an internal structure of the treatment tool attachment-assisting instrument 90.

As shown in FIGS. 17 and 18, the treatment tool attachment-assisting instrument 90 includes an assisting instrument main body 91, a discharge tube 92, and a plug body 93. The assisting instrument main body 91 has a tubular shape which can be fixed to the treatment tool channel port 103 of the endoscope apparatus 100. The discharge tube 92 communicates with the internal space of the assisting instrument main body 91. The plug body 93 is disposed on an extension line extended along the center axis of the treatment tool channel 104 from the proximal opening of the treatment tool channel 104 in the treatment tool channel port 103.

The assisting instrument main body 91 has an attachment structure which can be water-tightly connected to the treatment tool channel port 103. The discharge tube 92 can be connected to a pipeline which is connected to a liquid-discharge container (not shown). The plug body 93 is a soft member which has an opening or a gap through which the plug body 93 can come into close contact with the outer circumferential surface 3c of the sheath 3.

In the present embodiment, the treatment tool attachment-assisting instrument 90 is fixed to the treatment tool channel port 103 of the endoscope apparatus 100 before the treatment tool 1 for an endoscope is inserted into the treatment tool channel 104 (refer to FIG. 20). In the state where the treatment tool attachment-assisting instrument 90 is attached to the treatment tool channel port 103, liquid, which flows in reverse from the distal side of the treatment tool channel 104 of the endoscope apparatus 100 toward the proximal side thereof, mainly flows through the discharge tube 92. Accordingly, the liquid, which flows in reverse from the distal side of the treatment tool channel 104 of the endoscope apparatus 100 toward the proximal side thereof, is very unlikely to leak from the plug body 93 to the outside of the treatment tool attachment-assisting instrument 90.

Next, the operation of the treatment tool 1 for an endoscope according to the present embodiment will be described. In the present embodiment, an example is shown in which the treatment tool 1 for an endoscope according to the present embodiment is used along with the endoscope apparatus 100 in a case where endoscopic sphincterotomy (EST), Endoscopic retrograde cholangiopancreatography (ERCP), and calculus removal are sequentially performed as a series of procedures.

FIG. 19 is a view showing a process when the treatment tool 1 for an endoscope is used.

As shown in FIGS. 1 and 19, in the present embodiment, a side view type endoscope apparatus 100 which is suitable for observing a duodenal papilla PV is used.

For example, the side view type endoscope apparatus 100 includes a tubular member 101, a holding portion 102, a treatment tool channel port 103, a treatment tool channel 104, a raising stand 105, and an imaging portion 106. The tubular member 101 is a portion which is inserted into the body. The holding portion 102 is disposed at the proximal end of the tubular member 101. The treatment tool channel port 103 is disposed on a portion of the holding portion 102. The treatment tool channel 104 communicates with the treatment tool channel port 103 and is disposed at the inward side of the tubular member 101. The raising stand 105 is provided so as to be movable in an opening portion from which the treatment tool protrudes in order to change the direction of the treatment tool or the like protruding from the treatment tool channel 104 at the distal end 104a of the treatment tool channel 104 to the direction orthogonal to a center axis L8 of the tubular member 101. The imaging visual field of the imaging portion 106 faces the direction orthogonal to the center axis L8 of the tubular member 101. The imaging portion 106 is provided so as to be adjacent to the opening portion from which the treatment tool protrudes.

The treatment tool 1 for an endoscope according to the present embodiment can be suitably used for both of an aspect in which an operator of the endoscope apparatus 100 and an operator of the treatment tool 1 for an endoscope are different from each other, and an aspect in which the hook 46 is connected to the holding portion 102 of the endoscope apparatus 100 such that one operator operates the endoscope apparatus 100 and the treatment tool 1 for an endoscope.

The case where the treatment tool 1 for an endoscope is used will be described.

First, in a state where the treatment tool 1 for an endoscope is not attached to the endoscope apparatus 100, as shown in FIG. 19, according to the known procedure, an operator guides the endoscope apparatus 100 to the duodenal papilla PV which is the target portion to be treated and observes the target portion to be treated using the endoscope apparatus 100. At this time, the treatment tool attachment-assisting instrument 90 may be attached to the treatment tool channel port 103, and may not be attached to the treatment tool channel port 103.

FIG. 20 is a view when the state where the treatment tool 1 for an endoscope is attached to the endoscope apparatus 100 is viewed from a viewpoint of an operator of the endoscope apparatus 100.

As shown in FIG. 20, after the target portion to be treated is observed, the hook 46 is attached to the holding portion 102 of the endoscope apparatus 100 in a state where the treatment tool attachment-assisting instrument 90 is fixed to the treatment tool channel port 103. In addition, the sheath 3 of the treatment tool 1 for an endoscope according to the present embodiment is inserted into the treatment tool channel 104 (refer to FIG. 1) of the endoscope apparatus 100 via the treatment tool attachment-assisting instrument 90. The operation portion 40 of the treatment tool 1 for an endoscope is connected to the endoscope apparatus 100 via the hook 46. Accordingly, for example, an operator of the treatment tool 1 for an endoscope can extract and input the sheath 3 with respect to the treatment tool channel port 103 of the endoscope apparatus 100 in a state of holding the endoscope apparatus 100 by the left hand and the sheath 3 by the right hand.

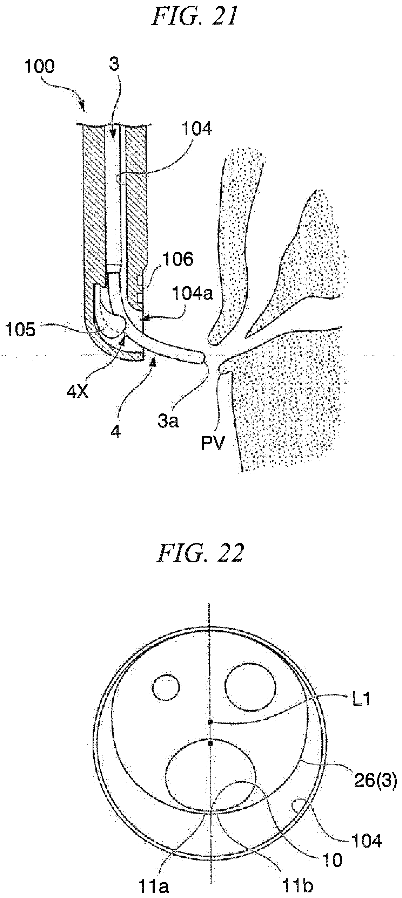

FIG. 21 is a view showing a process in which the treatment tool 1 for an endoscope is used. FIG. 22 is a sectional view showing a state where a soft region of the sheath 3 is positioned on the inner surface of a treatment tool channel at the bendable portion. FIG. 23 is a schematic view showing the treatment tool 1 for an endoscope according to the present embodiment which is reflected on an endoscopic image which is imaged using the endoscope apparatus 100.

As shown in FIG. 21, the sheath 3 is operated by an operation, the distal end 3a of the sheath 3 protrudes from the distal end 104a (opening portion) of the treatment tool channel 104, and as shown in FIG. 23, imaging is performed by the imaging portion 106 of the endoscope apparatus 100.

In a procedure of the EST with respect to the duodenal papilla PV using the side view type endoscope apparatus 100, in a case where the image captured by the endoscope apparatus 100 is viewed from the viewpoint of a dial plate of a timepiece in which the upper center of the image is set to twelve o'clock, the direction of the imaging portion is adjusted such that the incision target portion of the duodenal papilla PV is reflected between eleven o'clock and twelve o'clock in the image captured by the endoscope apparatus 100. In this state, by incising the duodenal papilla PV such that the duodenal papilla PV is expanded from the opening portion of the duodenal papilla PV, a passage through which a calculus or the like in the duodenal papilla PV passes is formed.

The operator causes the high-rigidity region 25 to pass through the treatment tool channel 104 which is curved by the bendable portion 107 of the endoscope apparatus 100. At this time, since the curved shape is applied to the high-rigidity region 25 in advance, until the curved shape of the pre-curved portion 4 follows the curved shape of the treatment tool channel 104 inside the treatment tool channel 104 curved by the bendable portion 107 or the raising stand 105, the pre-curved portion 4 including the high-rigidity region 25 is passively rotated with the center axis L1 of the sheath 3 as a rotation center. In the present embodiment, since the proximal slit portion 10p is formed, the proximal side region is more easily twisted relative to the proximal end of the pre-curved portion 4. Accordingly, when the high-rigidity region 25 of the pre-curved portion 4 is passively rotated in the treatment tool channel 104 so as to follow the curved shape of the treatment tool channel 104 curved by the bendable portion 107 of the endoscope apparatus 100, a rotation resistance of the proximal side region can be further decreased relative to the high-rigidity region 25.

Thereafter, the operator causes the high-rigidity region 25 to protrude from the distal end 104a of the treatment tool channel 104 of the endoscope apparatus 100.

Subsequently, the operator causes the high-rigidity region 25 including the drawing portion 5 to enter an imaging visual field of the imaging portion 106 of the endoscope apparatus 100. Since the rotation resistance of the proximal side region further decreases relative to the high-rigidity region 25, after the high-rigidity region 25 protrudes from the distal end 104a of the treatment tool channel 104 of the endoscope apparatus 100, if the high-rigidity region 25 including the drawing portion 5 moves forward as it is, the cutting portion 34 protruding from the first distal communication hole 23 and the second distal communication hole 24 easily protrudes in the direction closer to an eleven o'clock direction than a twelve o'clock direction. Accordingly, in a case where the treatment tool 1 for an endoscope according to the present embodiment is applied to the side view type endoscope apparatus 100 to incise the duodenal papilla PV, it is possible to perform the incision at a position at which an amount of bleeding is small. In addition, if the proximal end 3b of the sheath 3 is not positively rotated with the center axis L1 of the sheath 3 as the rotation center, since the high-rigidity region 25 is passively rotated according to the bending state of the bendable portion 107 or the raising stand 105 of the endoscope apparatus 100, the distal end 3 a of the sheath 3 is curved in the twelve o'clock direction in the endoscopic image.

In the state where the high-rigidity region 25 including the drawing portion 5 enters the imaging visual field of the imaging portion 106, as shown in FIG. 21, the outer circumferential surface of the copying-deformation portion 6 of the pre-curved portion 4 is pressed by the raising stand 105. In the outer circumferential surface of the copying-deformation portion 6 of the pre-curved portion 4, the position of a pressed surface 4x which is pressed by the raising stand 105 is the position on the outer circumferential surface of the copying-deformation portion 6 of the pre-curved portion 4.

In the state where the high-rigidity region 25 including the drawing portion 5 enters the imaging visual field of the imaging portion 106, the soft region 26 is positioned at the portion at which the treatment tool channel 104 is bent by the bendable portion 107 of the endoscope apparatus 100. At this time, as shown in FIG. 22, the copying-deformation portion 6 is pressed by the inner surface of the treatment tool channel 104 which is bent by the bendable portion 107. Since the distal slit portion 10d is formed in the copying-deformation portion 6, if an external force is applied to the outer circumferential surface of the copying-deformation portion, the contour shape of the copying-deformation portion 6 in the cross section orthogonal to the center axis L1 of the sheath 3 is easily deformed. Specifically, since the distal slit portion 10d is formed, resistance decreases due to the deformation generated when the contour shape of the copying-deformation portion 6 is to be deformed in an elliptical shape. Accordingly, if the outer circumferential surface of the copying-deformation portion 6 is pressed by the inner surface of the treatment tool channel 104, the contour shape of the copying-deformation portion 6 becomes a slightly elliptical shape.

If the shape of the copying-deformation portion 6 becomes a slightly elliptical shape, the copying-deformation portion 6 is not easily rotated in the treatment tool channel 104 which is bent by the bendable portion 107 of the endoscope apparatus 100. Accordingly, the state where the cutting portion 34 protruding from the first distal communication hole 23 and the second distal communication hole 24 protrudes in the direction closer to the eleven o'clock direction than the twelve o'clock direction is easily maintained. This effect is remarkable in a case where the tissues are subjected to an external force such as a reaction force.

As shown in FIG. 23, when the distal portion of the sheath 3 is displayed on the endoscopic image by the imaging portion 106 and the curved direction of the pre-curved portion 4 on the endoscopic image is set to twelve o'clock, the cutting portion 34 protrudes from the first distal communication hole 23 and the second distal communication hole 24 in the direction closer to the eleven o'clock direction relative to the twelve o'clock direction. The operator advances the sheath 3 in the treatment tool channel 104 while confirming the cutting portion 34 being directed in the direction between eleven o'clock and twelve o'clock on the endoscopic image. Accordingly, the distal end 3 a of the sheath 3 reaches the opening of the duodenal papilla PV and is inserted into the duodenal papilla PV.

In addition, in a case where the determination of the position at which the distal end 3a of the sheath 3 is inserted into the duodenal papilla PV is difficult, the operator may insert the guide wire 80 from the first port 49 of the operation portion 40 into the first lumen 7 so as to allow the distal end 80a of the guide wire 80 to protrude from the distal end 7a of the first lumen 7. In this case, first, after the operator inserts the distal end 80a of the guide wire 80 into the duodenal papilla PV, subsequently, the operator can insert the distal end 3 a of the sheath 3 into the duodenal papilla PV along the guide wire 80.

After the sheath 3 is introduced into the duodenal papilla PV, the operator may inject a contrast agent from the second port 62 so as to introduce the contrast agent into the bile duct through the duodenal papilla PV from the distal discharge port 17 of the second lumen 15 of the sheath 3. According to the introduction of the contrast agent, the operator can easily recognize traveling of the bile duct, presence or absence, the position, and the size of a calculus, or the like.

After the contrast agent is introduced, in a case where removal of the calculus is required, the EST is performed.

FIG. 24 is a schematic view showing the endoscopic image in a process of treatment using the treatment tool 1 for an endoscope.

As shown in FIG. 24, after the sheath 3 is introduced into the duodenal papilla PV, in a state where the distal portion (drawing portion 5) of the sheath 3 is disposed in the duodenal papilla PV by a predetermined length, the slider portion 71 of the operation portion 40 moves from the direction of the proximal end 68b of the shaft portion 68 in the direction of the center axis of the rod-shaped portion 69 of the shaft portion 68, that is, the direction of the center axis L5 (refer to FIG. 2) of the handle-fixing portion 64. Accordingly, the knife wire 30 moves in the direction of the proximal end of the knife wire 30, and the distal end 30a of the knife wire 30 generates a force which moves the portion of the first distal communication hole 23 of the sheath 3 in the proximal direction. Therefore, the proximal portion of the distal end 3 a of the sheath 3 is deformed to be curved between the first distal communication hole 23 and the second distal communication hole 24. In addition, the cutting portion 34 of the knife wire 30 is suspended in an arch shape with respect to the sheath 3.

The curved knife portion 35 (refer to FIG. 12A) is positioned at the second quadrant Q2 in the above-described virtual coordinate system. Accordingly, when the curved direction of the pre-curved portion 4 which is imaged as the endoscopic image by the imaging portion 106 is set to twelve o'clock, the curved knife portion 35 comes into contact with the opening portion of the duodenal papilla PV at the position which is biased so as to be closer to the eleven o'clock direction relative to the twelve o'clock direction.

In the process in which the curved knife portion 35 is suspended in an arch shape with respect to the sheath 3, the operator supplies a high-frequency current from a high-frequency power supply device to the knife wire 30 through the connector 73 of the operation portion 40. Accordingly, the tissues which come into contact with the curved knife portion 35 are incised by the high-frequency current. The curved knife portion 35 is curved in a natural state where external force is not applied to the curved knife portion 35. Since the knife wire 30 is moved in the direction of the proximal end 68b of the shaft portion 68 by the slider portion 71, the curved knife portion 35 is gradually deformed from the curved shape in the natural state into a straight-line shape. Specifically, the curved knife portion 35 is gradually deformed from the curvedt shape in the natural state into the straight-line shape along the straight-line direction in which the first distal communication hole 23 and the second distal communication hole 24 are connected to each other. If the curvature radius of the pre-curved portion 4 is set to be large, the knife wire 30 is gradually deformed from a straight-line shape into a curved shape. In this way, the curved state of the curved knife portion 35 is changed by the movement of the knife wire 30 which uses the slider portion 71.

Since the position of the duodenal papilla PV avoiding a main blood vessel is set so as to be reflected from eleven o'clock on the endoscopic image and the duodenal papilla PV is incised by the curved knife portion 35 at the position of eleven o'clock, it is possible to perform incision in a state where the amount of bleeding due to the incision of the duodenal papilla PV decreases.

After the incision with respect to the duodenal papilla PV ends, if necessary, the operator connects a syringe which is filled with the contrast agent to the second port 62 and injects the contrast agent from the second port 62 into the duodenal papilla PV through the second lumen 15. The path to the calculus which is the removal target is recognized on an X-line image by the contrast agent injected into the duodenal papilla PV.

In the state where the operation portion 40 is connected to the holding portion 102 of the endoscope apparatus 100 by the hook 46, the opening of the first port 49 faces the proximal side of the endoscope apparatus 100. Accordingly, an operator can insert the distal end of the guide wire 80 into the first lumen 7 in a state of viewing the first lumen 7 of the sheath 3 through the first port 49.

In addition, the first port 49 includes a long-hole shaped opening which is long in a longittudinal axis L2 direction extending in the straight-line direction in which the distal end 48a of the connection portion 48 to the sheath 3 and the proximal end 48b of the connection portion 48 to the sheath 3 are connected to each other. Accordingly, the guide wire 80 inserted into the first port 49 can be operated so as to move forward and backward in the direction (direction which is slightly inclined with the center axis L7 of the first lumen 7) along the approximately center axis L7 of the first lumen 7. Therefore, the operator operates the guide wire 80 so as to easily move the guide wire 80 forward and backward by the right hand, and it is possible to prevent the occurrence of buckling of the guide wire 80.

That is, according to the treatment tool 1 for an endoscope of the present embodiment, from the state where the operator holds the endoscope apparatus 100 by the left hand and moves the sheath 3 forward and backward by the right hand, the operator switches the holding target of the right hand from the sheath 3 to the guide wire 80 and can easily insert the guide wire 80 into the first port 49 or move the guide wire 80 forward and backward by the right hand.

In a case where the operator inserts the guide wire 80 into the first lumen 7 before the operator inserts the distal end 3 a of the sheath 3 into the duodenal papilla PV, the operator may use the guide wire 80 which has already been inserted, or may replace the inserted guide wire with the above-described angle type guide wire 80.

The operator causes the guide wire 80 to protrude from the distal end 3 a of the sheath 3, and guides the guide wire 80 to a desired position in the bile duct. At this time, if necessary, the operator may rotate the guide wire 80 around the center axis so as to move the guide wire 80 such that the distal end 80a of the guide wire 80 is inserted into a desired branch in a branching portion of the bile duct.

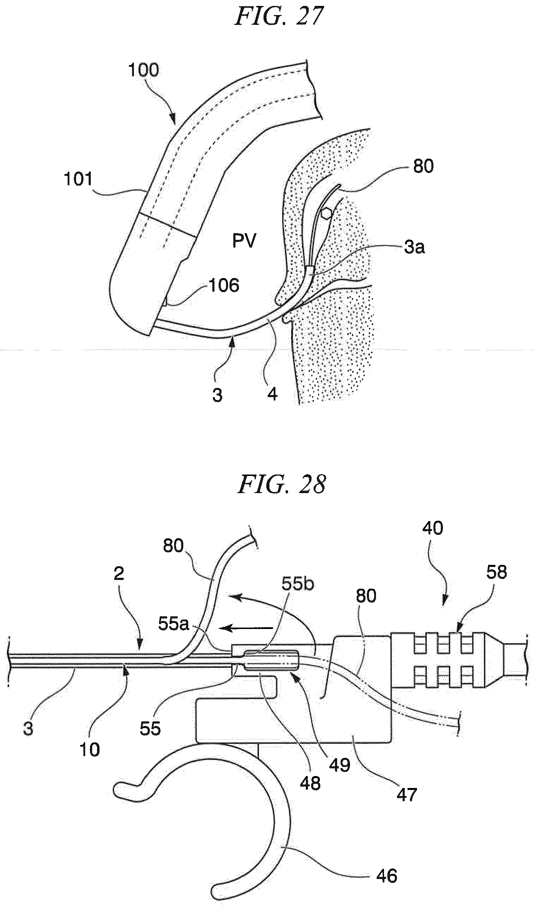

FIG. 27 is a view showing a process when the treatment tool 1 for an endoscope is used. As shown in FIG. 27, for example, the guide wire 80 is inserted until the distal end 80a of the guide wire 80 is positioned at the position advancing toward the inner side to a certain extent beyond the calculus to be removed from the bile duct.

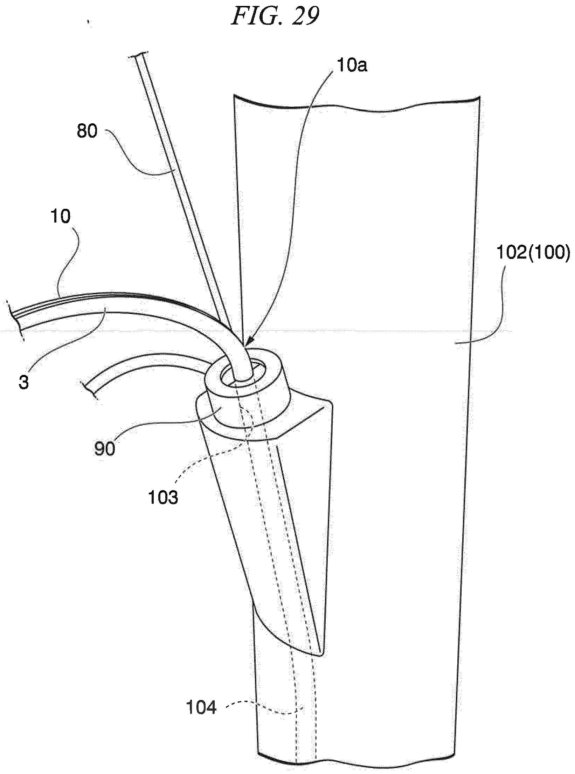

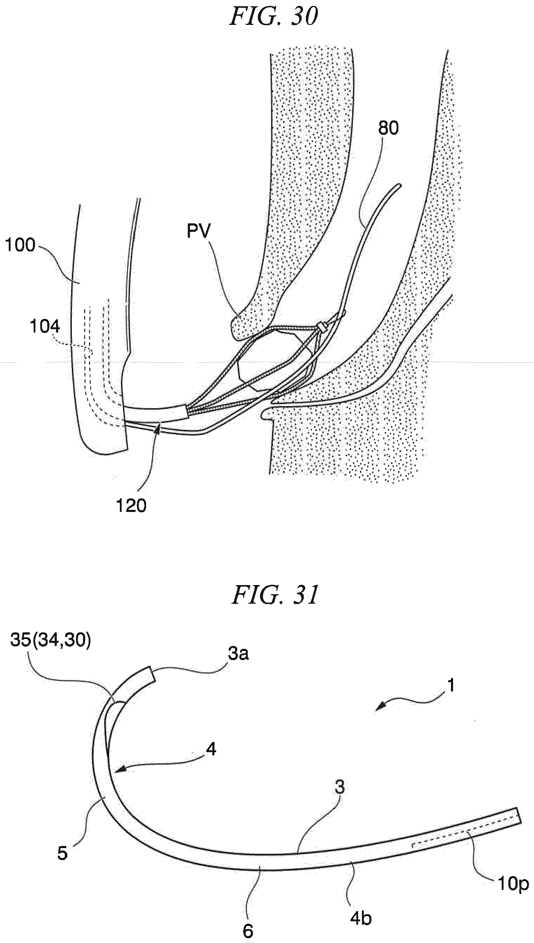

FIG. 28 is a view showing a process in which the treatment tool 1 for an endoscope is removed from the endoscope apparatus 100 in a state where the guide wire 80 which is attached to the treatment tool 1 for an endoscope remains. FIG. 29 is a view showing a process in which the sheath 3 and the guide wire 80 of the treatment tool 1 for an endoscope are spaced from each other. FIG. 30 is a view showing an example of treatment which is performed after the removal of the treatment tool 1 for an endoscope.

After the guide wire 80 reaches a predetermined position, the treatment tool 1 for an endoscope is removed in the state where the guide wire 80 remains in the body. This is performed in order to introduce the known endoscope calculus removal instrument (basket forceps, balloon, or the like) for removing the calculus into the bile duct instead of the treatment tool 1 for an endoscope according to the present embodiment.

As shown in FIG. 28, in order to remove the treatment tool 1 for an endoscope, first, the operator detaches the guide wire 80 from the first port 49, which is disposed in the distal configuration portion 41 of the operation portion 40, through the notch portion 55. At this time, the operator moves the guide wire 80 with respect to first port 49 from the proximal end 55b of the notch portion 55 of the first port 49 to the distal end 55a of the notch portion 55 through the inner portion of the notch portion 55 without changing the position of the distal end 80a of the guide wire 80. In the process in which the guide wire 80 passes through the notch portion 55 of the first port 49, the guide wire 80 is gradually extracted from the guide wire accommodation portion 9 to the outside of the sheath 3 through the slit portion 10.

Subsequently, as shown in FIG. 29, the operator moves the sheath 3 in the direction of the proximal end of the treatment tool channel 104 while supporting the guide wire 80 such that the position of the guide wire 80 is not changed. In the process in which the operator moves the sheath 3 in the direction of the proximal end of the treatment tool channel 104, the sheath 3 is gradually detached from the guide wire 80.

After the outlet portion 12 (refer to FIG. 10) of the first lumen 7 in the sheath 3 reaches the position of the treatment tool channel port 103, the operator moves the sheath 3 in the direction of the proximal end 80b of the guide wire 80 while supporting the guide wire 80 against the force which is generated by the guide wire 80 to be moved in the direction of the proximal end 80b. The operator extracts the outlet portion 12 of the sheath 3 from the treatment tool channel port 103 without changing the position of the guide wire 80 in the body. Thereafter, the operator moves the distal portion of the sheath 3, in which the outlet portion 12 of the sheath 3 is disposed, in the direction of the proximal end 80b of the guide wire 80, and detaches the sheath 3 from the guide wire 80.

After the sheath 3 is detached from the guide wire 80, the operator attaches the known endoscope calculus removal instrument (for example, basket forceps 120 shown in FIG. 30) to the guide wire 80, and guides the endoscope calculus removal instrument to the calculus which is the removal target through the treatment tool channel 104 of the endoscope apparatus 100.

Next, an example will be described in which an operator who operates the endoscope apparatus 100 and an operator who operates the treatment tool 1 for an endoscope are different from each other.

In this example, the operator who operates the treatment tool 1 for an endoscope holds the operation portion 40 of the treatment tool 1 for an endoscope by one hand, and can insert the guide wire 80 into the opening of the first port 49 and adjust the position of the guide wire 80 by the other hand. The operator of the endoscope apparatus 100 and the operator of the treatment tool 1 for an endoscope cooperate with each other with respect to mutual operations, and thus, it is possible to perform the same treatment as that of the above example in which one operator operates the endoscope apparatus 100 and the treatment tool 1 for an endoscope.

As described above, in the treatment tool 1 for an endoscope according to the present embodiment, since the first port 49 is disposed in the direction in which the inner surface 7c of the first lumen 7 is viewed through the opening of the first port 49 when the hook 46 is attached to the holding portion 102 of the endoscope apparatus 100, it is possible to easily insert the guide wire 80 into the opening of the first port 49 in a case where the hook 46 is attached to the holding portion 102 of the endoscope apparatus 100 and one person uses the endoscope apparatus 100 and the treatment tool 1 for an endoscope.

In addition, in the treatment tool 1 for an endoscope according to the present embodiment, since the first port 49 is disposed in the above-described direction, one operator can easily perform the forward and backward movements and the rotating operation of the guide wire 80 when the guide wire 80 is inserted from the opening of the first port 49 into the first lumen 7 with the holding portion 102 of the endoscope apparatus 100.

If the opening of the first port 49 is an elliptical opening, even when the distal end is an angle type guide wire 80 whose distal end is bent, it is possible to easily introduce the distal end into the first lumen 7.

In the present embodiment, in addition to the third lumen 20, the first lumen 7 which is the guide wire lumen and the second lumen 15 which is the liquid-feeding lumen are provided in the sheath 3. However, the first lumen and the second lumen are not essential constitutions. If at least the above-described first distal communication hole 23 and second distal communication hole 24 are formed at the third lumen, and the cutting portion 34 is inserted into the third lumen 20, the first distal communication hole 23, and the second distal communication hole 24 according to the above-described configuration, the present invention may be realized.

Hereafter, a first modification example of the treatment tool 1 for an endoscope according to the present embodiment will be described.

### (First Modification Example)

The first modification example of the first embodiment of the present invention will be described. FIG. 31 is a schematic view showing the configuration of the present modification example. As shown in FIG. 31, for example, the position of the distal end of the proximal slit portion 10p may be positioned at a position spaced from the proximal end 4b of the pre-curved portion 4. In this case, the distal slit portion 10d does not exist, or the distal end of the proximal slit portion 10p does not communicate with the distal slit portion 10d.

Hereinbefore, embodiments of the present invention are described. However, the technical scope of the present invention is not limited to the embodiments, combinations of components of the embodiments may be changed, various modifications may be applied to the components, and the components may be deleted within the scope which does not depart from the gist of the present invention. The present invention is not limited to the above-described explanations.

### [Industrial Applicability]

It is possible to provide a treatment tool for an endoscope and an incision system capable of stably incising a treatment portion while holding an incision instrument at a desired position.

### [Reference Signs List]

- 1:: treatment tool for an endoscope
- 2:: insertion portion
- 3:: sheath
- 3c:: outer circumferential surface
- 4:: pre-curved portion
- 7:: first lumen (guide wire lumen)
- 10:: slit portion
- 20:: third lumen (knife wire lumen)
- 23:: first communication hole
- 24:: second communication hole
- 34:: cutting portion
- 80:: guide wire
- 107:: bendable portion
- α:: first virtual plane (virtual plane)

## Claims

1. A treatment tool for an endoscope, comprising:
a sheath which has a center axis along a longitudinal axis;
a pre-curved portion which is disposed at a distal portion of the sheath, and has a restoring force to restore to a curved shape in which the sheath is curved along a virtual plane including the center axis of the sheath;
a knife wire lumen which has a center axis at a position spaced from the virtual plane at the distal portion of the pre-curved portion, and is formed along the center axis of the sheath;
a first communication hole which is open in a direction outwardly away from the position of the knife wire lumen in a radial direction with respect to the center axis of the sheath, and communicates an outer circumferential surface positioned at an inward side of the curved shape of the pre-curved portion with the knife wire lumen;
a second communication hole which is open in a direction outwardly away from the position of the knife wire lumen in the radial direction with respect to the center axis of the sheath, and communicates the outer circumferential surface positioned at the inward side of the curved shape of the pre-curved portion with the knife wire lumen at a more proximal position of the pre-curved portion than the first communication hole;
a wire-shaped cutting portion which protrudes from the first communication hole and the second communication hole and extends between the first communication hole and the second communication hole, the cutting portion being capable of incising tissues;
a fixing portion which is disposed at a distal end portion of the cutting portion, and disposed to fix the cutting portion and the knife wire lumen in a state that the fixing portion is inserted into the knife wire lumen;
a guide wire accommodation portion which is formed along a longitudinal axis of the sheath at a position spaced from the knife wire lumen in a circumferential direction around the center axis of the sheath, and into which a guide wire is capable of being inserted;
a proximal slit portion which is formed to communicate from the guide wire accommodation portion to the outer circumferential surface of the sheath in a region more proximal than a proximal end of the pre-curved portion;
a distal slit portion which is formed to communicate an outer circumferential surface at an outward side of the curved shape of the pre-curved portion with the guide wire accommodation portion from the proximal end of the pre-curved portion to an intermediate portion of the pre-curved portion in a direction toward a distal end side of the pre-curved portion; and
a high-rigidity region which extends from a distal end of the sheath to a distal end of the distal slit portion, and has a higher torsional rigidity than that of a region more proximal than the proximal end of the pre-curved portion.

2. The treatment tool for an endoscope according to Claim 1,
wherein an inlet portion is formed such that an inner portion of the guide wire accommodation portion is communicated with the outer circumferential surface of the sheath at a proximal end side of the sheath, the inlet portion being capable of being inserted by that the guide wire, and
wherein the proximal slit portion is continuously formed from the proximal end of the pre-curved portion to the inlet portion.

3. A treatment tool for an endoscope, comprising:
a sheath which has a center axis along a longitudinal axis;
a pre-curved portion which is disposed at a distal portion of the sheath, and has a restoring force to restore to a curved shape in which the sheath is curved along a virtual plane including the center axis of the sheath;
a knife wire lumen which has a center axis at a position spaced from the virtual plane at the distal portion of the pre-curved portion, and is formed along the center axis of the sheath;
a first communication hole which is open in a direction outwardly away from the position of the knife wire lumen in a radial direction with respect to the center axis of the sheath, and communicates an outer circumferential surface positioned at an inward side of the curved shape of the pre-curved portion with the knife wire lumen;
a second communication hole which is open in a direction outwardly away from the position of the knife wire lumen in the radial direction with respect to the center axis of the sheath, and communicates the outer circumferential surface positioned at the inward side of the curved shape of the pre-curved portion with the knife wire lumen at a more proximal position of the pre-curved portion than the first communication hole;
a wire-shaped cutting portion which protrudes from the first communication hole and the second communication hole and extends between the first communication hole and the second communication hole, the cutting portion being capable of incising tissues;
a fixing portion which is provided at a distal end portion of the cutting portion, and provided to fix the cutting portion and the knife wire lumen in a state that the fixing portion is inserted into the knife wire lumen;
a guide wire accommodation portion which is formed along a longitudinal axis of the sheath at a position spaced from the knife wire lumen in a circumferential direction around the center axis of the sheath, and into which a guide wire is capable of being inserted; and
a distal slit portion which is formed along the center axis of the sheath such that an outer circumferential surface at an outward side of the curved shape of the pre-curved portion is communicated with the guide wire accommodation portion from the proximal end of the pre-curved portion to an intermediate portion of the pre-curved portion in a direction toward a distal end side of the pre-curved portion.

4. The treatment tool for an endoscope according to Claim 3,
wherein a proximal region of the pre-curved portion including the distal slit portion has a rigidity such that the proximal region of the pre-curved portion including the distal slit portion is deformed to cause a contour shape of the proximal region of the pre-curved portion including the distal slit portion to be changed from a circular shape to an elliptical shape in a cross section orthogonal to the center axis of the sheath, the proximal region of the pre-curved portion including the distal slit portion being deformed by receiving a force from an inner wall of a treatment tool channel which is bent by a bendable of the endoscope apparatus.
